(19) Europäisches Patentamt
European Patent Office
Office européen des brevets

(11) EP 4 393 520 A1

(12) **EUROPEAN PATENT APPLICATION**
published in accordance with Art. 153(4) EPC

(43) Date of publication:
**03.07.2024 Bulletin 2024/27**

(21) Application number: **22861382.4**

(22) Date of filing: **24.08.2022**

(51) International Patent Classification (IPC):
***A61L 27/20** (2006.01)* ***A61L 27/38** (2006.01)*
***A61L 27/52** (2006.01)*

(52) Cooperative Patent Classification (CPC):
**A61L 27/20; A61L 27/38; A61L 27/52**

(86) International application number:
**PCT/JP2022/031816**

(87) International publication number:
**WO 2023/027094 (02.03.2023 Gazette 2023/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.08.2021 JP 2021136866**

(71) Applicants:
- **Mochida Pharmaceutical Co., Ltd.**
  **Tokyo 160-8515 (JP)**
- **National University Corporation Hokkaido University**
  **Sapporo-shi, Hokkaido 060-0808 (JP)**
- **Konan Gakuen**
  **Kobe shi, Hyogo 658-8501 (JP)**

(72) Inventors:
- **IWASAKI, Norimasa**
  **Sapporo-shi, Hokkaido 060-0808 (JP)**
- **ONODERA, Tomohiro**
  **Sapporo-shi, Hokkaido 060-0808 (JP)**
- **YAMAGUCHI, Jun**
  **Tokyo 105-8461 (JP)**
- **NAGAHAMA, Koji**
  **Kobe-shi, Hyogo 650-0047 (JP)**
- **SAITO, Mitsuru**
  **Tokyo 105-8461 (JP)**

(74) Representative: **Hoffmann Eitle**
**Patent- und Rechtsanwälte PartmbB**
**Arabellastraße 30**
**81925 München (DE)**

(54) **TREATED CELL MATERIAL USED IN BIOLOGICAL TISSUE REPAIR**

(57) It is desirable to provide a novel biomaterial and the like for tendon repair. Provided is a biomaterial for tendon repair containing a hydrogel formed by means of a cross-linking reaction between a reactive group A and a reactive group B, wherein the hydrogel contains a water-soluble polymer having the reactive group A, and mesenchymal stem cells having the reactive group B.

FIG. 1

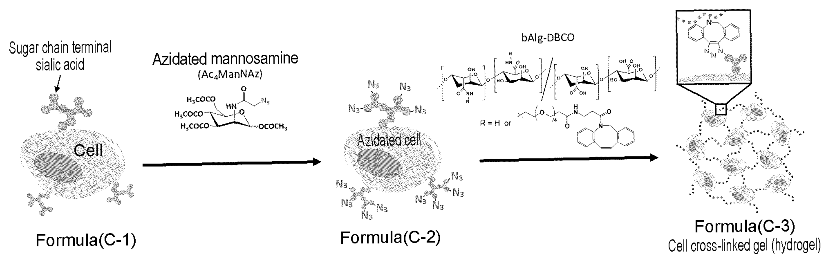


Formula(C-1)

Formula(C-2)

Formula(C-3)
Cell cross-linked gel (hydrogel)

EP 4 393 520 A1

## Description

[Technical Field]

The present invention relates to a biological material for tendon repair that exhibits a gel state by the chemical binding of a water-soluble polymer with mesenchymal stem cells as cross-linking points and exerts an excellent tendon repair effect, a method for producing the biological material for tendon repair, a kit for tendon repair, and the like.

[Background Art]

Hydrogels are soft materials in which a three-dimensional network of a polymer is swollen in water, and are widely used in, for example, food, cosmetic, houseware, and environmental fields because of having characteristics such as water absorbability, substance retention, substance release, and substance separation. The hydrogels further have unique characteristics such as flexibility, elasticity, biocompatibility, and cell retention characteristics. Therefore, the development of their application is expected to medical-related fields which require high performance, such as sensors, diagnostic devices, drug delivery systems, and regenerative medicine.

For example, a technique of using cells having the ability to regenerate tissues in the repair of injured tissues has received attention, and various studies have been made on composite materials made of cells incorporated in gels. Patent Literature 1 has reported that a composite material made of cells incorporated in a hydrogel comprising a temperature-responsive polymer having water solubility and biodegradability and a clay mineral having a nanosheet structure is effective for inured tissue repair. However, such a conventional composite material made of cells incorporated in a gel, when administered to injured tissues, may be incapable of exerting a sufficient tissue regeneration effect due to the cells or growth factors produced by the cells are eliminated to the outside of the gel because the cells are merely physically supported within the gel. Thus, there has been a demand for further improvement.

In recent years, there has been a growing need for treatment methods for the atrophy of muscular tissues as the population ages. Particularly, the muscle of the lower extremities has been found to define the exercise tolerability of heart failure patients. The maintenance of muscular strength is essential for the extension of not only health prognosis but vital prognosis. For example, a methods using a gel material for transplantation chemically cross-linked to myoblasts or muscle satellite cells have been proposed as such treatment methods and reportedly produced favorable regeneration of muscular tissues (see Non Patent Literatures 1 and 2 and Patent Literatures 2 to 4). Also, Non Patent Literature 3 discloses a method for repairing a tendon tissue using mesenchymal stem cells.

[Citation List]

[Non Patent Literature]

Non Patent Literature 1: Nature communications, 2018, Vol. 9, Article No. 2195, pp. 1-11

Non Patent Literature 2: Advanced Biology, 2021, Vol. 5, Article No. 2000106.

Non Patent Literature 3: Journal of orthopaedic research, 2017 Feb; 35 (2): 269-280


[Patent Literature]

Patent Literature 1: Japanese Patent Laid-Open No. 2015-40276

Patent Literature 2: Japanese Patent Laid-Open No. 2020-192021

Patent Literature 3: U.S. Patent Publication No. 2020/0405914

Patent Literature 4: International Publication No. WO 2020/241904


[Summary of Invention]

[Technical Problem]

Under these circumstances, there has been a demand for providing a novel a biological material for tendon repair, or the like.

[Solution to Problem]

The present inventors have conducted diligent studies and consequently found that when a hydrogel formed through the chemical binding of a water-soluble polymer with mesenchymal stem cells as cross-linking points is administered to an injured tendon tissue, the cells remain in the hydrogel at an affected part and are capable of effectively repairing the tendon tissue. The present invention has been completed through further studies based on this finding.

Specifically, the following is provided herein.

[1] A biological material for tendon repair, comprising a hydrogel formed through cross-linking reaction of a reactive group A and a reactive group B, wherein the hydrogel comprises:

a water-soluble polymer having the reactive group A; and
mesenchymal stem cells having the reactive group B.

[2A] The material according to [1], wherein a combination of the reactive group A and the reactive group B is selected from the group consisting of combinations in the following table:

[Table 1]

| | Reactive group A | Reactive group B |
|---|---|---|
| RG-1 | Alkynyl group | Azide group |
| RG-2 | Azide group | Alkynyl group |
| RG-3 | Carboxyl group | Amino group |
| RG-4 | N-Hydroxysuccinimide ester group | Amino group |
| RG-5 | N-Succinimide group | Amino group |
| RG-6 | Amino group | Carboxyl group |
| RG-7 | Amino group | N-Hydroxysuccinimide ester group |
| RG-8 | Amino group | N-Succinimide group |
| RG-9 | Sulfhydryl group | Sulfhydryl group |
| RG-10 | Sulfhydryl group | Pyridyl disulfide group |
| RG-11 | Sulfhydryl group | Maleimide group |
| RG-12 | Pyridyl disulfide group | Sulfhydryl group |
| RG-13 | Maleimide group | Sulfhydryl group |

[2B] The material according to [1] or [2A], wherein the reactive group A is an alkynyl group, and the reactive group B is an azide group.

[3] The material according to any one of [1] to [2B], wherein the reactive group A is a cyclic alkynyl group, and the reactive group B is an azide group.

[4] The material according to any one of [1] to [3], wherein the water-soluble polymer comprises alginic acid bound with a branched compound.

[4A] The material according to any one of [1] to [4], wherein the water-soluble polymer comprises alginic acid bound with a branched compound selected from the group consisting of the following formulas (1-N), (1-N-1), (1-N-2-1), and (1-N-2-2):

[Table 2]

| | |
|---|---|
| Formula (1-N) | 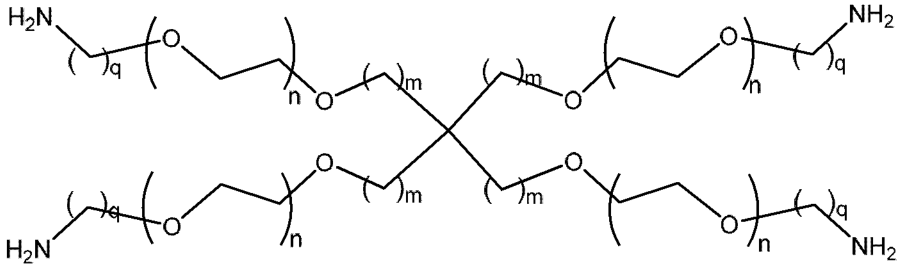 In formula (1-N), m = 1 to 10, n = 10 to 500, and q = 1 to 10 |
| Formula (1-N-1) | In formula (1-N-1), $n^1$ is, for example, 10 to 500, preferably 10 to 300, more preferably 10 to 200 |
| Formula (1-N-2-1) | |
| | 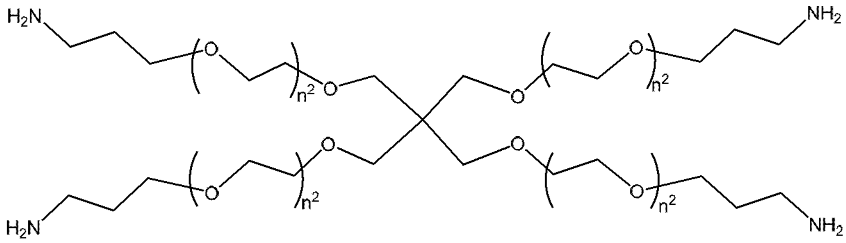 In formula (1-N-2-1), $n^2$ is, for example, 10 to 500, preferably 10 to 300, more preferably 10 to 200 |
| Formula (1-N-2-2) | 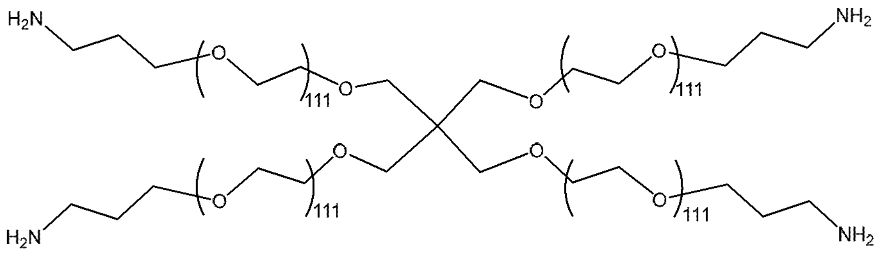 |

[4B] The material according to any one of [1] to [4A], wherein the water-soluble polymer comprises branched alginate (bAlg) represented by the following formula:

[Chem. 1]

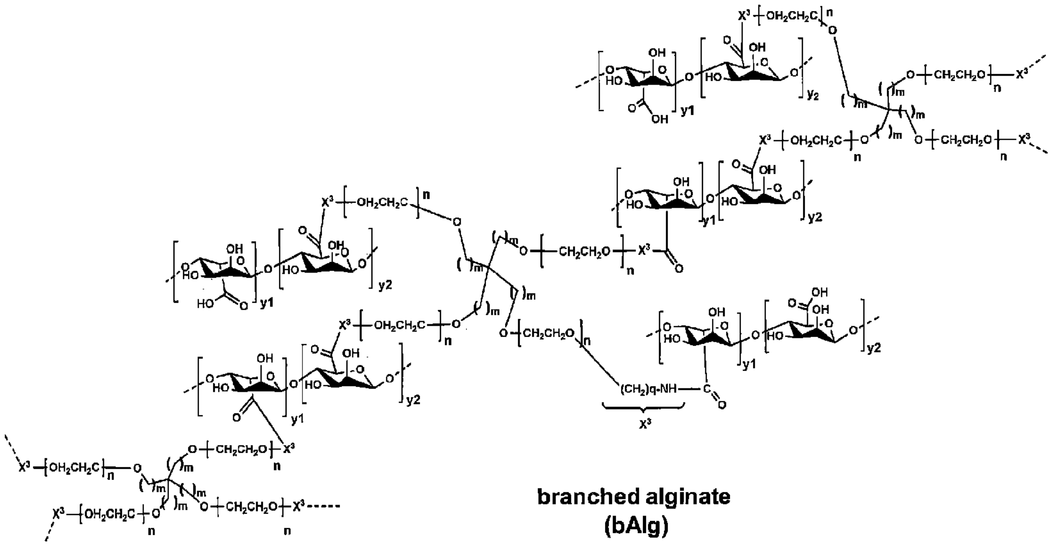

branched alginate
(bAlg)

wherein m = 1 to 10, n = 10 to 500, and q = 1 to 10;
$y_1$ and $y_2$ are values obtained according to the following calculation expressions (when each of the values is a value with a decimal point, an integer value after rounding down to the nearest decimal or an integer value after rounding up to the whole number is accepted):

$$(1)\ y_1 = (MW_{ALGN} - 18.01) / 198.11(1 + MG),$$

and

$$(2)\ y_2 = MG \times y_1$$

wherein $MW_{ALGN}$ is a molecular weight (weight-average molecular weight) of sodium alginate to be bound to the branched compound; and MG is a M/G ratio (= $y_2/y_1$) of sodium alginate to be bound to the branched compound; and $X^3$ = $-NH(CH_2)_q-$ is a binding pattern in which the imino group (-NH-) is bonded through an amide bond to a carboxyl group of alginate.

[4B-1] In [4B], m is preferably 1 to 5, more preferably 1.
[4B-2] In [4B], n is preferably 10 to 300, more preferably 10 to 200, further preferably 111.
[4B-3] In [4B], q is preferably 1 to 5, more preferably 3.
[4B-4] In [4B], $y_1$ and $y_2$ are values obtained according to the following calculation expressions (when each of the values is a value with a decimal point, an integer value after rounding down to the nearest decimal or an integer value after rounding up to the whole number is accepted):

$$(1)\ y_1 = (MW_{ALGN} - 18.01) / 198.11(1 + MG),$$

and

$$(2)\ y_2 = MG \times y_1$$

wherein $MW_{ALGN}$ is a molecular weight (weight-average molecular weight) of sodium alginate to be bound to the branched compound and is in the range of, for example, 10,000 to 10,000,000, preferably 100,000 to 5,000,000, more preferably 150,000 to 3,000,000; and

MG is a M/G ratio (= $y_2/y_1$) of sodium alginate to be bound to the branched compound and is in the range of, for example, 0.1 to 4.0, 0.1 to 3.0, 0.1 to 2.0, 0.5 to 1.8, 0.8 to 1.2, or 0.1 to 0.5, and

each of $y_1$ and $y_2$ is preferably 253.

[4B-5] In [4B-4], $MW_{ALGN}$ and MG are a molecular weight (weight-average molecular weight) of sodium alginate and a M/G ratio of sodium alginate described in Table 3.

[4C] The material according to any one of [1] to [4A], wherein the water-soluble polymer comprises branched alginate (bAlg) represented by the following formula:

[Chem. 2]

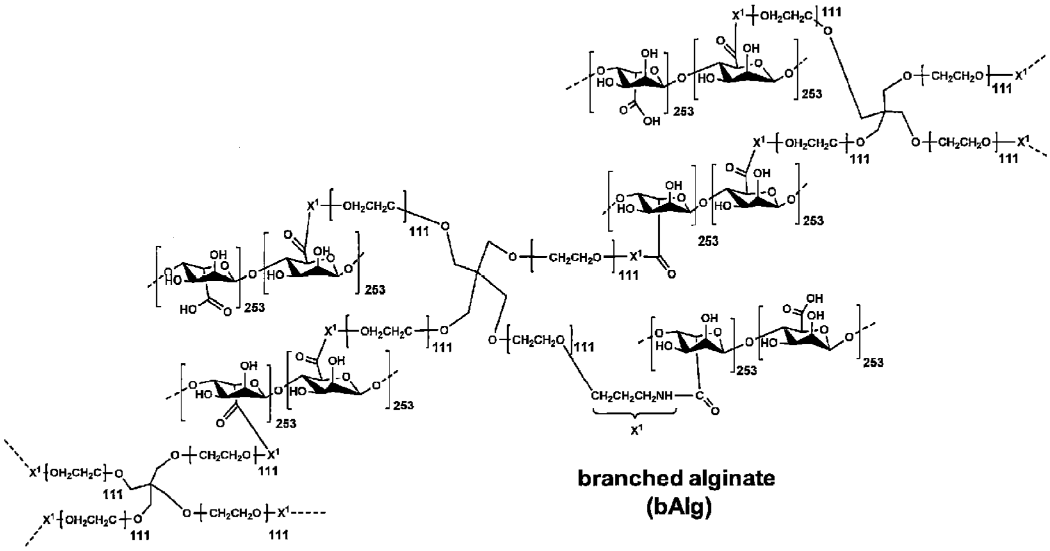

branched alginate (bAlg)

wherein $X^1$ = $-NHCH_2CH_2CH_2-$ is a binding pattern in which the imino group (-NH-) is bonded through an amide bond to a carboxyl group of alginate.

[4-1] The material according to any one of [1] to [4C], wherein the water-soluble polymer having the reactive group A comprises cyclooctyne-modified branched alginate (bAlg-DBCO) represented by the following formula:

[Chem. 3]

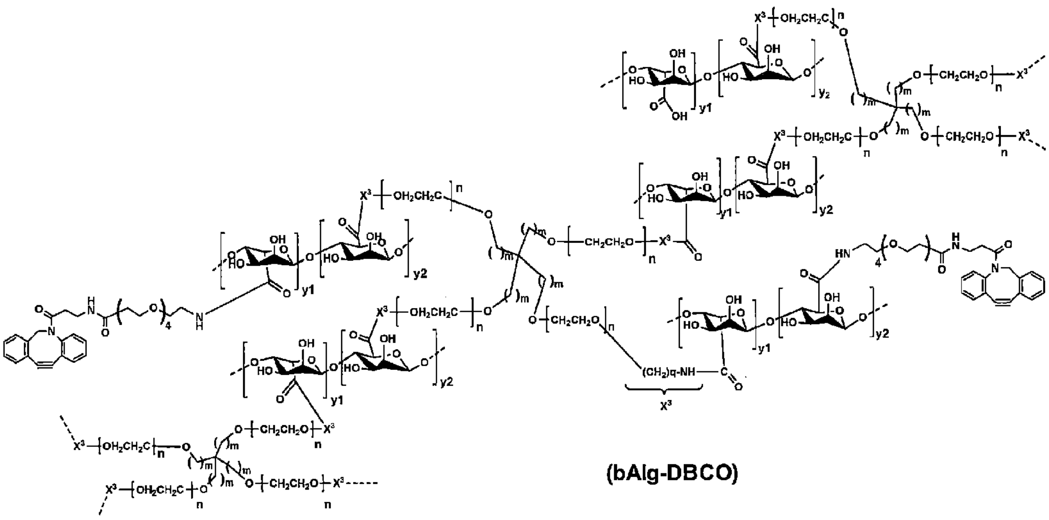

(bAlg-DBCO)

wherein m = 1 to 10, n = 10 to 500, and q = 1 to 10;
$y_1$ and $y_2$ are values obtained according to the following calculation expressions (when each of the values is a value with a decimal point, an integer value after rounding down to the nearest decimal or an integer value after rounding up to the whole number is accepted):

$$(1)\ y_1 = (MW_{ALGN} - 18.01) / 198.11(1 + MG),$$

and

$$(2)\ y_2 = MG \times y_1$$

wherein $MW_{ALGN}$ is a molecular weight (weight-average molecular weight) of sodium alginate to be bound to the branched compound; and MG is a M/G ratio (= $y_2/y_1$) of sodium alginate to be bound to the branched compound; and $X^3$ = $-NH(CH_2)_q-$ is a binding pattern in which the imino group (-NH-) is bonded through an amide bond to a carboxyl group of alginate.

[4-1-1] In [4-1], m is preferably 1 to 5, more preferably 1.
[4-1-2] In [4-1], n is preferably 10 to 300, more preferably 10 to 200, further preferably 111.
[4-1-3] In [4-1], q is preferably 1 to 5, more preferably 3.
[4-1-4] In [4-1], $y_1$ and $y_2$ are values obtained according to the following calculation expressions (when each of the values is a value with a decimal point, an integer value after rounding down to the nearest decimal or an integer value after rounding up to the whole number is accepted):

$$(1)\ y_1 = (MW_{ALGN} - 18.01) / 198.11(1 + MG),$$

and

$$(2)\ y_2 = MG \times y_1$$

wherein $MW_{ALGN}$ is a molecular weight (weight-average molecular weight) of sodium alginate to be bound to the branched compound and is in the range of, for example, 10,000 to 10,000,000, preferably 100,000 to 5,000,000, more preferably 150,000 to 3,000,000; and
MG is a M/G ratio (= $y_2/y_1$) of sodium alginate to be bound to the branched compound and is in the range of, for example, 0.1 to 4.0, 0.1 to 3.0, 0.1 to 2.0, 0.5 to 1.8, 0.8 to 1.2, or 0.1 to 0.5, and

each of $y_1$ and $y_2$ is preferably 253.

[4-1-5] In [4-1-4], $MW_{ALGN}$ and MG are a molecular weight (weight-average molecular weight) of sodium alginate and a M/G ratio of sodium alginate described in Table 3.

[4-2] The material according to any one of [1] to [4C], wherein the water-soluble polymer having the reactive group A comprises cyclooctyne-modified branched alginate (bAlg-DBCO) represented by the following formula: wherein $X^1$ = $-NHCH_2CH_2CH_2-$ is a binding pattern in which the imino group (-NH-) is bonded through an amide bond to a carboxyl group of alginate.

[5] The material according to any one of [1] to [4-2], wherein the mesenchymal stem cells are derived from bone marrow.

[6] The material according to any one of [1] to [5], wherein the number of cells contained in the hydrogel is approximately $1.0 \times 10^5$ cells/mL or more.

[6-1] The material according to any one of [1] to [5], wherein the number of cells contained in the hydrogel is approximately $1.0 \times 10^7$ to approximately $2.0 \times 10^8$ cells/mL.

[7] The material according to any one of [1] to [6-1], wherein a concentration of the water-soluble polymer is approximately 0.2% by weight to approximately 8.0% by weight based on the total amount of the hydrogel.

[7-1] The material according to any one of [1] to [6-1], wherein a concentration of the water-soluble polymer is approximately 2.0% by weight to approximately 4.0% by weight based on the total amount of the hydrogel.

[8] The material according to any one of [1] to [7-1], wherein the hydrogel is an injectable gel.

[9] The material according to any one of [1] to [8], wherein the material is used in the repair of a tendon tissue selected from the group consisting of patella tendon, tibialis anterior tendon, Achilles tendon, popliteal tendon, semitendinosus tendon, gracilis tendon, abductor tendon, adductor tendon, supraspinatus tendon, infraspinatus tendon, subscapularis tendon, teres minor tendon, flexor tendon, rectus femoris tendon, posterior tibial tendon, and quadriceps femoris tendon.

[9-1] The material according to any one of [1] to [8], wherein the material is used in the repair of a tendon tissue selected from the group consisting of patella tendon, tibialis anterior tendon, Achilles tendon, popliteus tendon, semitendinosus tendon, gracilis tendon, supraspinatus tendon, infraspinatus tendon, subscapularis tendon, teres minor tendon, rectus femoris tendon, posterior tibial tendon, and quadriceps femoris tendon.

[10] A method for producing a biological material for tendon repair, comprising the step of subjecting a water-soluble polymer having a reactive group A and mesenchymal stem cells having a reactive group B to cross-linking reaction.

[10-1] The method for producing a biological material for tendon repair according to [10], wherein a combination of the reactive group A and the reactive group B is selected from the group consisting of combinations in the table described in [2A].

[10-2] The method for producing a biological material for tendon repair according to [10] or [10-1], wherein the reactive group A is an alkynyl group, and the reactive group B is an azide group.

[10-3] The method for producing a biological material for tendon repair according to any one of [10] to [10-2], wherein the reactive group A is a cyclic alkyne group, and the reactive group B is an azide group.

[10-4] The method for producing a biological material for tendon repair according to any one of [10] to [10-3], wherein the water-soluble polymer comprises alginic acid bound with a branched compound.

[10-5] The method for producing a biological material for tendon repair according to any one of [10] to [10-4], wherein the water-soluble polymer comprises alginic acid bound with a branched compound selected from the group consisting of the formulas (1-N), (1-N-1), (1-N-2-1), and (1-N-2-2) described in [4A].

[10-6] The method for producing a biological material for tendon repair according to any one of [10] to [10-5], wherein the water-soluble polymer comprises the branched alginate (bAlg) described in [4B] or [4C].

[11] The method for producing a biological material for tendon repair according to any one of [10] to [10-6], wherein the water-soluble polymer having the reactive group A is the cyclooctyne-modified branched alginate (bAlg-DBCO) described in [4-1] or [4-2], and the reactive group B is an azide group.

[12] A kit for tendon repair, comprising:

a water-soluble polymer having a reactive group A; and
mesenchymal stem cells having a reactive group B, wherein
the kit is used such that a hydrogel obtained through cross-linking reaction of the reactive group A and the reactive group B is applied.

[12-1] The kit for tendon repair according to [12], wherein a combination of the reactive group A and the reactive group B is selected from the group consisting of combinations in the table described in [2A].

[12-2] The kit for tendon repair according to [12] or [12-1], wherein the reactive group A is an alkynyl group, and the reactive group B is an azide group.

[12-3] The kit for tendon repair according to any one of [12] to [12-2], wherein the reactive group A is a cyclic alkyne group, and the reactive group B is an azide group.

[12-4] The kit for tendon repair according to any one of [12] to [12-3], wherein the water-soluble polymer comprises alginic acid bound with a branched compound.

[12-5] The kit for tendon repair according to any one of [12] to [12-4], wherein the water-soluble polymer comprises alginic acid bound with a branched compound selected from the group consisting of the formulas (1-N), (1-N-1), (1-N-2-1), and (1-N-2-2) described in [4A].

[12-6] The kit for tendon repair according to any one of [12] to [12-5], wherein the water-soluble polymer comprises the branched alginate (bAlg) described in [4B] or [4C].

[12-7] The kit for tendon repair according to any one of [12] to [12-6], wherein the water-soluble polymer having the reactive group A is the cyclooctyne-modified branched alginate (bAlg-DBCO) described in [4-1] or [4-2], and the reactive group B is an azide group.

[13] A composition for use in tendon repair, comprising a water-soluble polymer having a reactive group A.

[13-1] The composition for use in tendon repair according to [13], wherein the reactive group A is selected from the group consisting of an alkynyl group, an azide group, a carboxyl group, a N-hydroxysuccinimide ester group, a N-succinimide group, an amino group, a sulfhydryl group, a pyridyl disulfide group, and a maleimide group.

[13-2] The composition for use in tendon repair according to [13] or [13-1], wherein the reactive group A is an alkynyl group.

[13-3] The composition for use in tendon repair according to any one of [13] to [13-2], wherein the reactive group A is a cyclic alkyne group.

[13-4] The composition for use in tendon repair according to any one of [13] to [13-3], wherein the water-soluble polymer comprises alginic acid bound with a branched compound.

[13-5] The composition for use in tendon repair according to any one of [13] to [13-4], wherein the water-soluble polymer comprises alginic acid bound with a branched compound selected from the group consisting of the formulas (1-N), (1-N-1), (1-N-2-1), and (1-N-2-2) described in [4A].

[13-6] The composition for use in tendon repair according to any one of [13] to [13-5], wherein the water-soluble polymer comprises the branched alginate (bAlg) described in [4B] or [4C].

[14] A composition for use in tendon repair, comprising a water-soluble polymer having a reactive group A and mesenchymal stem cells having a reactive group B.

[15] A composition for use in tendon repair, comprising a hydrogel formed through cross-linking reaction of a water-soluble polymer having a reactive group A and mesenchymal stem cells having a reactive group B.

[16-1] The composition for use in tendon repair according to [14] or [15], wherein a combination of the reactive group A and the reactive group B is selected from the group consisting of combinations in the table described in [2A].

[16-2] The composition for use in tendon repair according to any one of [14] to [16-1], wherein the reactive group A is an alkynyl group, and the reactive group B is an azide group.

[16-3] The composition for use in tendon repair according to any one of [14] to [16-2], wherein the reactive group A is a cyclic alkyne group, and the reactive group B is an azide group.

[16-4] The composition for use in tendon repair according to any one of [14] to [16-3], wherein the water-soluble polymer comprises alginic acid bound with a branched compound.

[16-5] The composition for use in tendon repair according to any one of [14] to [16-4], wherein the water-soluble polymer comprises alginic acid bound with a branched compound selected from the group consisting of the formulas (1-N), (1-N-1), (1-N-2-1), and (1-N-2-2) described in [4A].

[16-6] The composition for use in tendon repair according to any one of [14] to [16-5], wherein the water-soluble polymer comprises the branched alginate (bAlg) described in [4B] or [4C].

[16-7] The composition for use in tendon repair according to any one of [14] to [16-6], wherein the water-soluble polymer having the reactive group A is the cyclooctyne-modified branched alginate (bAlg-DBCO) described in [4-1] or [4-2], and the reactive group B is an azide group.

[Advantageous Effects of Invention]

The present invention provides a novel biological material for tendon repair, or the like. In the biological material according to one aspect of the present invention, mesenchymal stem cells are bound with a water-soluble polymer through a chemical bond in a hydrogel. Therefore, even when the biological material is administered to a tendon tissue having injury, the cells can be maintained in the hydrogel and remain at an affected part, and can effectively repair the injured tendon tissue. In a preferred aspect, the biological material of the present invention has significantly better orientation of cells and tendon fibers at an injured site based on a modified Watkins score on 4 weeks after transplantation, as compared with an untreated group. Furthermore, the biological material of the present invention has the clinical advantages that the biological material allows for cell differentiation and tissue repair adapted to the environment by the application of a suitable mechanical load (stretch stimulation), and in addition, allows for low invasive transdermal cell transplantation. Moreover, the biological material according to one aspect of the present invention employs alginic acid and is therefore expected to have effects such as release of suitable humoral factors (e.g., cytokines and chemokines)

from transplanted cells and mitigation of immune response (prevention of fibrosis and scarring).

[Brief Description of Drawings]

[Figure 1] Figure 1 is a schematic view of a process of preparing a cell cross-linked gel.
[Figure 2] Figure 2 is a schematic view showing a prepared injured part of Achilles tendon of a Lewis rat.
[Figure 3] Figure 3 is photographs of an injured part of Achilles tendon on postoperative weeks 2 and 4 of each group.
[Figure 4] Figure 4 is a diagram showing results of histological evaluation on postoperative week 2 of each group.
[Figure 5] Figure 5 is a diagram showing results of histological evaluation on postoperative week 2 of each group (PLM).
[Figure 6] Figure 6 is a diagram showing a modified Watkins score on postoperative week 2 of each group.
[Figure 7] Figure 7 is a diagram showing results of histological evaluation on postoperative week 4 of each group.
[Figure 8] Figure 8 is a diagram showing results of histological evaluation on postoperative week 4 of each group (PLM).
[Figure 9] Figure 9 is a diagram showing a modified Watkins score on postoperative week 4 of each group.
[Figure 10] Figure 10 is a diagram showing results of qPCR of each group.
[Figure 11] Figure 11 is a diagram showing results of a stretch test of each group.

[Description of Embodiments]

The biological material provided herein is used for the purpose of repairing a tendon tissue. The biological material according to one aspect of the present invention is a biological material for tendon repair, comprising a hydrogel formed through cross-linking reaction of a reactive group A and a reactive group B, wherein the hydrogel comprises: a water-soluble polymer having the reactive group A; and mesenchymal stem cells having the reactive group B. Hereinafter, the biological material of the present invention will be described in detail.

[Water-soluble polymer]

In one aspect of the present invention, the biological material comprises a water-soluble polymer having the reactive group A as a base material for forming the hydrogel.

The water-soluble polymer can be of type having biocompatibility and being capable of forming the hydrogel when covalently bound with cells as cross-linking points. Specific examples of the water-soluble polymer include: polysaccharide thickeners such as alginic acid, chondroitin, pectin, gellan gum, carrageenan, curdlan, hyaluronic acid, and agarose; proteins such as collagen, gelatin, and fibrin; and synthetic polymers such as polyethylene glycol, MPC (2-methacryloyloxyethyl phosphorylcholine) polymers, and block copolymers thereof. The water-soluble polymer is preferably a polysaccharide thickener, more preferably alginic acid.

The alginic acid described in the present specification means at least one alginic acid (also referred to as "alginic acid, etc.") selected from the group consisting of alginic acid, alginic acid ester, and salts thereof (e.g., sodium alginate). The alginic acid used may be naturally derived or may be a synthetic product, and is preferably naturally derived.

The alginic acid is a natural polysaccharide that is extracted, purified, and produced from seaweed of brown algae, and is a polymer obtained by polymerizing D-mannuronic acid (M) and L-guluronic acid (G). The constitutional ratio of D-mannuronic acid and L-guluronic acid (M/G ratio), i.e., gel strength, of the alginic acid differs depending mainly on the type of an organism of origin such as seaweed and is also influenced by the habitat of the organism or season. The M/G ratio has a broad range from approximately 0.2 (high-G type) to approximately 5 (high-M type). The alginic acid differs in physicochemical properties and may differ in preferred purpose, depending on the M/G ratio of the alginic acid, a manner in which M and G are arranged, etc. The gelling ability of the alginic acid, etc. and the properties of a formed gel are influenced by the M/G ratio. In general, gel strength is known to be high when the proportion of G is high. The M/G ratio also additionally influences the hardness, fragility, water absorbability, flexibility, and the like of the gel. Thus, the alginic acid used in the present invention can have a suitable M/G ratio or a suitable viscosity according to its final intended use.

An industrial method for producing the alginic acid includes an acid method and a calcium method. In the present invention, alginic acid produced by any production method can be used. Its quantitative value based on HPLC by purification preferably falls within the range of 80% by mass to 120% by mass, more preferably within the range of 90% by mass to 110% by mass, further preferably within the range of 95% by mass to 105% by mass. In the present invention, the alginic acid whose quantitative value based on HPLC falls within the range described above is referred to as ultra purified alginic acid (UPAL). The alginic acid or the salt thereof used in the present invention is not particularly

limited and is preferably ultra purified alginic acid (UPAL). A commercially available product, for example, sold by Wako Pure Chemical Corp., Sigma-Aldrich Co., LLC, or MOCHIDA PHARMACEUTICAL CO., LTD.., preferably of ultra purified grade for a food or a medicament, can be purchased and used. The commercially available product may be further appropriately purified for use. For example, low endotoxin treatment is preferred. Methods for the purification or the low endotoxin treatment can adopt methods described in, for example, Japanese Patent Laid-Open No. 2007-75425. In the present specification, the term "% by mass" means w/w% or w/v%.

The alginic acid salt of the "alginic acid" used in the present invention is a "monovalent metal salt of the alginic acid" and is a salt that is produced by the ion exchange of a hydrogen ion of carboxylic acid of D-mannuronic acid or L-guluronic acid of the alginic acid with a monovalent metal ion such as $Na^+$ or $K^+$. Specific examples of the monovalent metal salt of the alginic acid can include sodium alginate and potassium alginate. Particularly, sodium alginate is preferred.

The alginic acid used in the present invention has a suitable weight-average molecular weight according to its intended use. The weight-average molecular weight (GPC) of the alginic acid used in the present invention is, for example, 10,000 to 10,000,000, preferably 100,000 to 5,000,000, more preferably 150,000 to 3,000,000.

In some aspects, the alginic acid is sodium alginate. The sodium alginate used can be a commercially available product of sodium alginate. In this context, sodium alginate sold by, for example, Wako Pure Chemical Corp., Sigma-Aldrich Co., LLC, or MOCHIDA PHARMACEUTICAL CO., LTD.. can be used as sodium alginate used in Examples mentioned later. In particular, sodium alginate manufactured by MOCHIDA PHARMACEUTICAL CO., LTD.. is UPAL(R). Specifically, sodium alginate of UPAL 20, UPAL 100, UPAL500, UPAL 20B, UPAL 100B, or UPAL 500B described in the table given below can be used. The viscosity, weight-average molecular weight, and M/G ratio of a 1 w/w% aqueous solution of each sodium alginate are shown in the following table.

[Table 3]

| Sodium alginate | Viscosity of 1 w/w% (mPa·s) | Weight-average molecular weight | | M/G ratio |
|---|---|---|---|---|
| | | GPC | GPC-MALS | |
| UPAL 20 | 10 to 40 | 300,000 to 700,000 | 60,000 to 130,000 | 0.5 to 1.8 |
| UPAL 100 | 50 to 150 | 700,000 to 1,400,000 | 130,000 to 200,000 | |
| UPAL 500 | 300 to 600 | 1,400,000 to 2,000,000 | 200,000 to 400,000 | |
| UPAL 20B | 10 to 40 | 150,000 to 800,000 | 60,000 to 130,000 | 0.1 to 0.5 |
| UPAL 100B | 70 to 150 | 800,000 to 1,500,000 | 130,000 to 200,000 | |
| UPAL 500B | 400 to 600 | 1,500,000 to 2,500,000 | 200,000 to 350,000 | |

The M/G ratio of the alginic acid (sodium alginate) can be measured in accordance with a known method (method described in Carbohydrate Research, 32 (1974), 217-225). The alginic acid (sodium alginate) differs in resistance to hydrolysis and solubility in an acid among a MM fraction, a GG fraction, and a MG fraction mentioned later. Accordingly, the alginic acid (sodium alginate) is degraded under conditions that cause cleavage into a MM fraction, a GG fraction, and a MG fraction using weak hydrochloric acid, and then fractionated into these fractions in accordance with a routine method. Subsequently, the sugar content of each fraction is measured by a phenol sulfate method, and the M/G ratio can be calculated according to the following expression:

M/G ratio = (Sugar content of the MM fraction + (Sugar content of the MG fraction / 2)) / (Sugar content of the GG fraction + (Sugar content of the MG fraction / 2))

The alginic acid is a linear polysaccharide constituted by D-mannuronic acid (M) and L-guluronic acid (G) and is a complicated block copolymer in which a homopolymer fraction of mannuronic acid (MM fraction):

[Chem. 5]

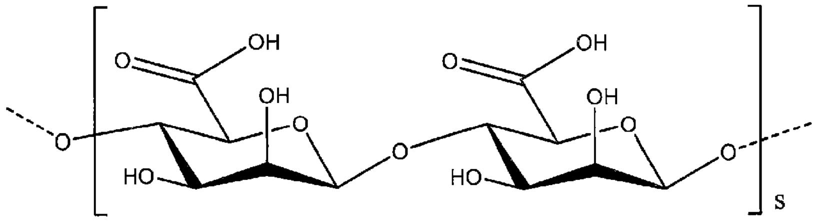

D-mannuronic acid D-mannuronic acid

wherein s is an arbitrary integer,
a homopolymer fraction of guluronic acid (GG fraction):

[Chem. 6]

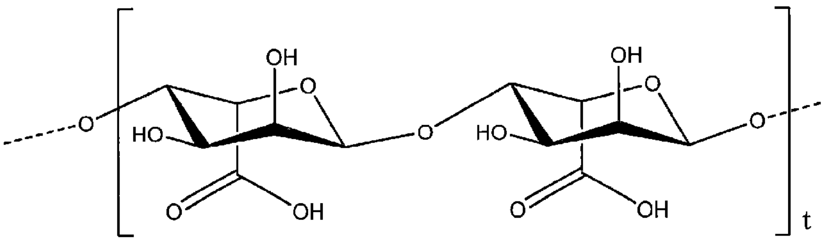

L-guluronic acid L-guluronic acid

wherein t is an arbitrary integer, and
a randomly arranged fraction of mannuronic acid and guluronic acid (MG fraction):

[Chem. 7]

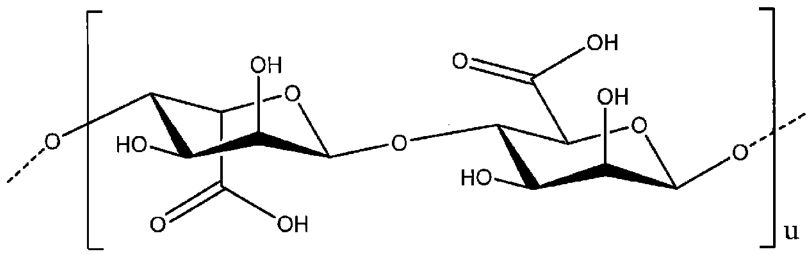

L-guluronic acid D-mannuronic acid

wherein u is an arbitrary integer,
are arbitrarily bound. Therefore, the structural formulas of the alginic acid and the monovalent metal salt of the alginic acid may be formally represented by, for example, the following formula (ALG-1):

[Chem. 8]

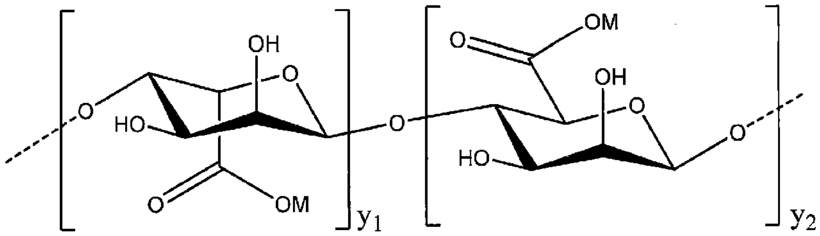

(ALG-1)

L-guluronic acid D-mannuronic acid

wherein each of $y_1$ and $y_2$ is an arbitrary integer; M is a hydrogen or a monovalent metal (e.g., potassium or sodium), and both terminal hydrogen atoms may be omitted (see Hydrogels Fibers (Published: August 1st, 2018), DOI:

10.5772/intechopen.74188), and
the following formula (ALG-2):

[Chem. 9]

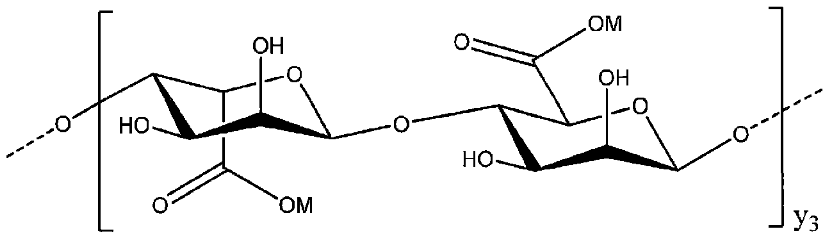

(ALG-2)

wherein $y_3$ is an arbitrary integer; and M is a hydrogen or a monovalent metal (e.g., potassium or sodium).

When the alginic acid or the monovalent metal salt of the alginic acid is represented by the formula (ALG-1), $y_1$ and $y_2$ in this formula can be calculated using the molecular weight (weight-average molecular weight: GPC or GPC-MALS) and M/G ratio of the alginic acid or the monovalent metal salt of the alginic acid used.
For example, $y_{1N}$ and $y_{2N}$ of sodium alginate of the following formula (ALG-1-N):

[Chem. 10]

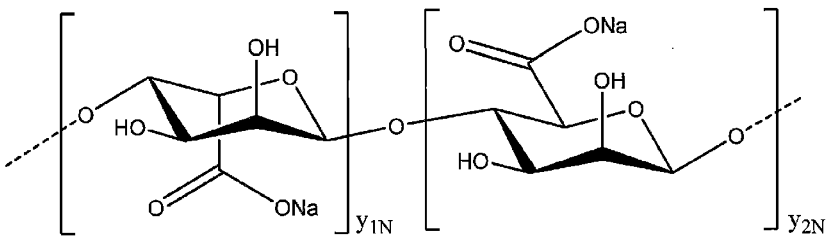

(ALG-1-N)

$MW_{GLA}$=198.11 $MW_{MNA}$=198.11

(molecular weight = approximately 10,000, and M/G ratio (= $y_{2N}/y_{1N}$) = 1.0) is calculated to be $y_{1N} = y_{2N}$ = 252.3 according to the following calculation expressions (1) and (2) (each of $y_{1N}$ and $y_{2N}$ is an integer and can therefore be 252 or 253).

(1) Molecular weight of sodium alginate ($MW_{ALGN}$): approximately 100,000 = Molecular weight of a sodium L-guluronate unit ($MW_{GLA}$ = 198.11) × $y_{1N}$ + Molecular weight of a sodium D-mannuronate ($MW_{MNA}$ = 198.11) × $y_{1N}$ + 18.01 (molecular weight of a $H_2O$ moiety that is not contained in each unit), and
(2) $y_{1N} = y_{2N}$

For example, when each sodium alginate having a distinctive molecular weight and M/G ratio is represented by the formula (ALG-1), its $y_1$ and $y_2$ can be calculated according to the following calculation expressions (when each of the obtained $y_1$ and $y_2$ is a numeric value with a decimal point, an integer value after rounding down to the nearest decimal or an integer value after rounding up to the whole number is accepted).

$$(3)\ y_1 = (MW_{ALGN} - 18.01) / 198.11(1 + MG),$$

and

$$(4)\ y_2 = MG \times y_1$$

wherein $MW_{ALGN}$ is the molecular weight (weight-average molecular weight) of sodium alginate; and MG is the M/G ratio (= $y_2/y_1$) of sodium alginate.

Methods for measuring the physical property values (viscosity measurement, weight-average molecular weight measurement, etc.) of each sodium alginate described in the table above and bAlg or bAlg-DBCO in the present specification are not particularly limited, and these physical property values can be measured by, for example, various methods known in literatures (methods described in, for example, International Publication No. WO 2021-125255). Although the measurement methods are not limited to the methods known in literatures or the like, each physical property value may be different from those described above, depending on a measurement method.

In the present specification, the molecular weight of the alginic acid may be indicated by Da (Dalton) as a unit.

The constitutional ratio of D-mannuronic acid to L-guluronic acid (M/G ratio) of the alginic acid, etc. differs depending mainly on the type of an organism of origin such as seaweed and is also influenced by the habitat of the organism or season. The M/G ratio has a broad range from approximately 0.2 (high-G type) to approximately 5 (high-M type). The gelling ability of the alginic acid, etc. and the properties of a formed gel are influenced by the M/G ratio. In general, gel strength is known to be high when the proportion of G is high. The M/G ratio also additionally influences the hardness, fragility, water absorbability, flexibility, and the like of the gel. The M/G ratio of the alginic acid, etc. and/or the salt thereof used is usually 0.1 to 4.0 and is 0.1 to 3.0 in an aspect, 0.1 to 2.0 in an aspect, 0.5 to 1.8 in an aspect, or 0.8 to 1.2 in an aspect. In another aspect, the M/G ratio is 0.1 to 0.5.

The alginic acid used in the present invention has a suitable viscosity and a suitable M/G ratio according to its intended use.

In the present specification, a numeric range indicated by the term "to" refers to a range including numeric values described before and after "to" as the minimum and maximum values, respectively.

The alginic acid can assume the form of, for example, a monovalent salt of the alginic acid or a divalent salt of the alginic acid. Examples of the monovalent salt of the alginic acid include sodium alginate, potassium alginate, and ammonium alginate. The monovalent salt of the alginic acid is preferably sodium alginate or potassium alginate, more preferably sodium alginate. Examples of the divalent salt of the alginic acid include calcium alginate, magnesium alginate, barium alginate, and strontium alginate.

The alginic acid used in the present specification is alginic acid that has not undergone low endotoxin treatment in some aspects, or is alginic acid that has undergone low endotoxin treatment in other aspects. The low endotoxin means that the endotoxin level is so low that inflammation or heat generation is not substantially caused. More preferably, alginic acid, etc. that has undergone low endotoxin treatment is desirable.

A method for the low endotoxin treatment is not particularly limited, and this treatment can be performed by, for example, various methods known in literatures (methods described in, for example, International Publication No. WO 2021-125255).

The water-soluble polymer is preferably in a branched form bound with a branched compound having two or more (preferably (2 to 10) reactive groups (hereinafter, also referred to as a branching agent) in order to facilitate forming the hydrogel. In one aspect, the water-soluble polymer comprises alginic acid bound with a branched compound. In another aspect, the water-soluble polymer comprises cyclooctyne-modified branched alginate (bAlg-DBCO). bAlg-DBCO will be described in detail in Examples mentioned later. The type of the branching agent is not particularly limited as long as the branching agent is capable of linking the water-soluble polymer in a branched form. Preferred examples thereof include multi-arm polyethylene glycol derivatives. For example, two-arm, three-arm, four-arm, and eight-arm types are known about the multi-arm polyethylene glycol derivatives. Examples of the four-arm polyethylene glycol derivative for use as the branching agent include compounds having a structure represented by the following general formula (1):

[Chem. 11]

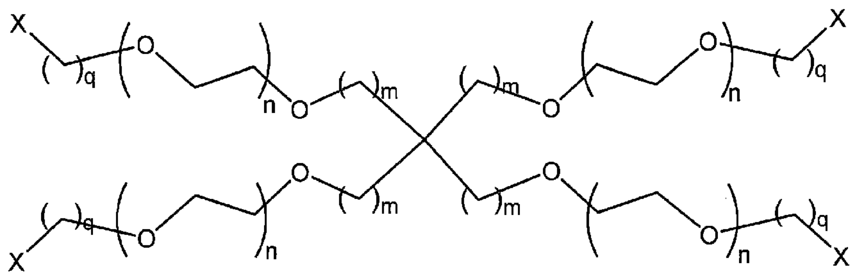

(1)

In the general formula (1), m is the number of methylene groups, for example, 1 to 10, preferably 1 to 5, more preferably 1.

In the general formula (1), n is an average number of moles of added ethylene oxide, for example, 10 to 500, preferably 10 to 300, more preferably 10 to 200.

In the general formula (1), q is the number of methylene groups, for example, 1 to 10, preferably 1 to 5, more preferably 3.

In the general formula (1), X is a reactive group capable of binding to a functional group of the water-soluble polymer to be bound. X (reactive group) can be appropriately set depending on the type of the functional group of the water-soluble polymer to be bound, and can be appropriately selected from, for example, a carboxyl group (-COOH), a N-succinimide group (-Suc), a N-hydroxysuccinimide ester group (-C(=O)-O-Suc), a sulfhydryl group (-SH), a pyridyl disulfide group (-S-S-(2-Pyr)), a maleimide group (-Mal), and an amino group (-$NH_2$). For example, when the water-soluble polymer to be bound has a carboxyl group, X (reactive group) can be an amino group. When the water-soluble polymer to be bound has an amino group, X (reactive group) can be selected from among a carboxyl group, a N-hydroxysuccinimide ester group, and a N-succinimide group. When the water-soluble polymer to be bound has a sulfhydryl group, X (reactive group) can be selected from among a sulfhydryl group, a pyridyl disulfide group, and a maleimide group.

Examples of the four-arm polyethylene glycol derivative include derivatives of the following formulas (1-N), (1-N-1), (1-N-2-1), and (1-N-2-2):

[Chem. 12]

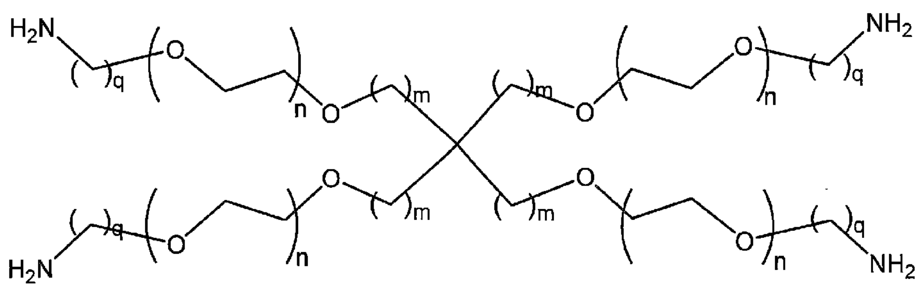

(1-N)

In the formula (1-N), m, n, and q are as defined in the formula (1).

[Chem. 13]

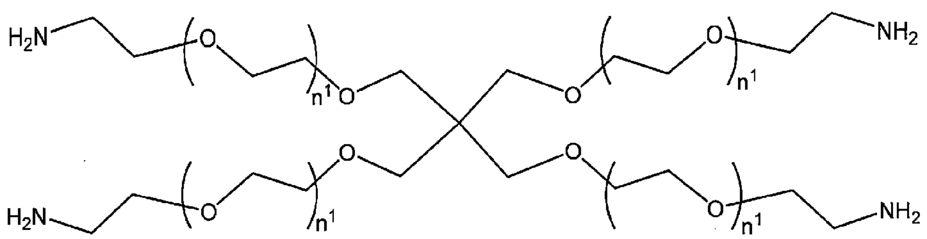

(1-N-1)

In the formula (1-N-1), $n^1$ is, for example, 10 to 500, preferably 10 to 300, more preferably 10 to 200.

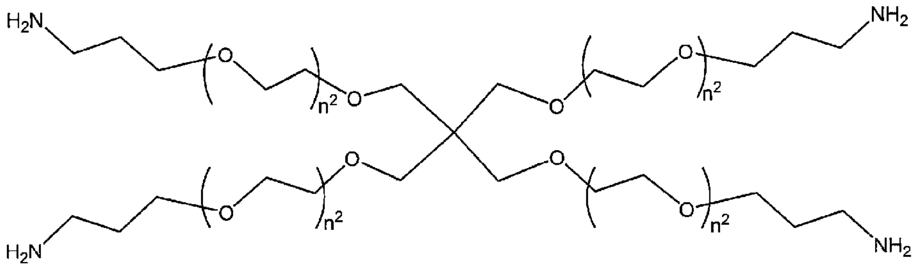

(1-N-2-1)

In the formula (1-N-2-1), $n^2$ is, for example, 10 to 500, preferably 10 to 300, more preferably 10 to 200.

[Chem. 15]

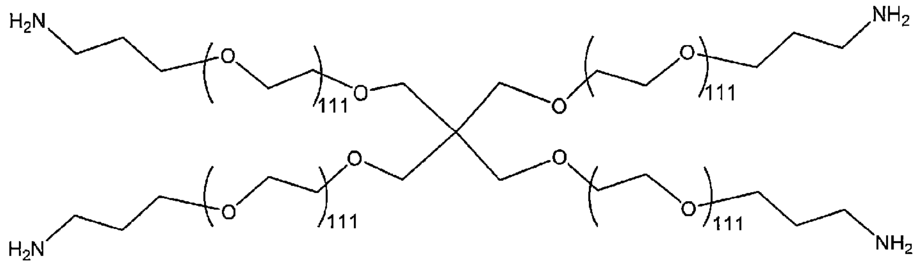

(1-N-2-2)

When the water-soluble polymer is prepared in a branched form by the binding of the branching agent, a reactive group of the branching agent can be bonded to a reactive group of the water-soluble polymer by a known approach.

When the branched water-soluble polymer is prepared by the binding of the branching agent, the ratio between the constituent water-soluble polymer and the branching agent can be appropriately set depending on the type or molecular weight of the constituent water-soluble polymer, the number of reactive groups of the branching agent, etc. The ratio can be, for example, 1 to 20 molecules, preferably 2 to 20 molecules, further preferably 2 to 10 molecules, of the branching agent per molecule of the constituent water-soluble polymer in the branched water-soluble polymer.

The water-soluble polymer has a reactive group A capable of being covalently bonded to a reactive group B of mesenchymal stem cells in order to form the hydrogel by chemically binding to the mesenchymal stem cells.

The type of the reactive group A can be appropriately set depending on the type of the reactive group B of the mesenchymal stem cells and can be appropriately selected from, for example, an alkynyl group, an azide group, a carboxyl group, an amino group, a N-succinimide group, a N-hydroxysuccinimide ester group, a sulfhydryl group, a pyridyl disulfide group, and a maleimide group. More specifically, when the reactive group B is an azide group, an alkynyl group can be selected as the reactive group A. When the reactive group B is an alkynyl group, an azide group can be selected as the reactive group A. When the reactive group B is a carboxyl group, a N-hydroxysuccinimide ester group, or a N-succinimide group, an amino group can be selected as the reactive group A. When the reactive group B is an amino group, the reactive group A can be selected from among a carboxyl group, a N-hydroxysuccinimide ester group, and a N-succinimide group. When the reactive group B is a sulfhydryl group, the reactive group A can be selected from among a sulfhydryl group, a pyridyl disulfide group, and a maleimide group. When the reactive group B is a pyridyl disulfide group, a sulfhydryl group can be selected as the reactive group A. When the reactive group B is a maleimide group, a sulfhydryl group can be selected as the reactive group A.

The reactive group A is preferably an alkynyl group or an azide group because the alkynyl group and the azide group can be chemically bonded conveniently by click chemistry. Specifically, preferred is a combination of an alkynyl group as the reactive group A introduced in the water-soluble polymer and an azide group as the reactive group B of the mesenchymal stem cells; or a combination of an azide group as the reactive group A introduced in the water-soluble polymer and an alkynyl group as the reactive group B of the mesenchymal stem cells. More preferred is a combination of an alkynyl group as the reactive group A introduced in the water-soluble polymer and an azide group as the reactive group B of the mesenchymal stem cells. In one aspect, the reactive group A is a cyclic alkyne group. The cyclic alkyne group is a 7- to 10-membered saturated hydrocarbon ring group, wherein one bond constituting the ring is a triple bond, preferably a cyclic alkyne group having an 8-membered ring. Examples of such a cyclic alkyne group include a cycloheptyne ring group, a cyclooctyne ring group, a cyclononyne ring group, and a cyclodecyne ring group. The cyclic alkyne group may be a cyclic alkyne group in which one to three methylene groups ($-CH_2-$) of the 7- to 10-membered saturated

hydrocarbon ring group are substituted by heteroatoms such as an oxygen atom, a NH group, a sulfur atom, and/or a sulfonyl group. Examples of such a cyclic alkyne group include a 4,5-didehydro-1,2,3,6,7,8-hexahydroazocine ring group. The cyclic alkyne group may be further condensed with one or two ring groups selected from a benzene ring, a 3- to 8-membered hydrocarbon ring, and an aromatic heterocyclic ring such as a pyridine ring. Examples of such a cyclic alkyne group include a bicyclo[6.1.0]non-4-yne ring group and a 11,12-dihydro-5,6-dihydro-dibenz[b,f]azocine ring group. In another aspect, examples of such a combination include a combination of bAlg-DBCO as the water-soluble polymer having the reactive group A and an azide group as the reactive group B.

Examples of the number of reactive groups A to be introduced to the water-soluble polymer include, but are not particularly limited to, 2 to 60, 5 to 50, or 5 to 30 reactive groups A per molecule of the water-soluble polymer.

For the introduction of the reactive group A to the water-soluble polymer, a bifunctional linker having the reactive group A and a reactive group C capable of binding to a functional group of the water-soluble polymer can be used to bond the reactive group C to the functional group of the water-soluble polymer. The reactive group C can be appropriately set depending on the type of the functional group of the water-soluble polymer to be bound, and can be appropriately selected from, for example, a carboxyl group, a N-succinimide group, a N-hydroxysuccinimide ester group, a sulfhydryl group, a pyridyl disulfide group, a maleimide group, and an amino group. For example, when the water-soluble polymer has a carboxyl group, the reactive group C can be an amino group. When the water-soluble polymer has an amino group, the reactive group C can be selected from among a carboxyl group, a N-hydroxysuccinimide ester group, and a N-succinimide group. When the water-soluble polymer has a sulfhydryl group, the reactive group C can be selected from among a sulfhydryl group, a pyridyl disulfide group, and a maleimide group.

The bifunctional linker having the reactive group A and the reactive group C can be a known bifunctional linker, or a bifunctional linker synthesized by a known approach can be used.

The present invention also relates to a composition for use in tendon repair, comprising a water-soluble polymer having the reactive group A mentioned above, or a composition for use in tendon repair, comprising a water-soluble polymer having the reactive group A and mesenchymal stem cells having the reactive group B. The features of the water-soluble polymer are as mentioned above. The tendon repair will be described later.

[Mesenchymal stem cell]

The biological material according to one aspect of the present invention comprises mesenchymal stem cells having a reactive group B capable of being covalently bonded to the reactive group A. The mesenchymal stem cells can have a reactive group B (e.g., an azide group) on the surface, as shown in Figure 1. The resulting cells themselves serve as cross-linking points of the water-soluble polymer and contribute to the formation of the hydrogel. The mesenchymal stem cells, one type of somatic stem cell, are stem cells that reside as stromal cells of mesenchymal tissues and have the ability to differentiate into multiple lineages and to self-renew, and are known to have the ability to differentiate into connective tissue cells such as osteocytes, chondrocytes, fat cells, myocardial cells, and nerve cells. The mesenchymal stem cells are cells that can differentiate into mesodermally derived fat or bone in the course of development and in addition, can be relatively easily obtained from bone marrow, fat tissues, dental pulp, or the like of an adult human, and have the ability to differentiate not only into mesodermal osteoblasts, fat cells, muscular cells, chondrocytes, and the like but into cells of endodermal visceral tissues, ectodermal nerves, and the like. Somatic stem cells include plural types such as neural stem cells and hematopoietic stem cells, in addition to the mesenchymal stem cells, and reside in various tissues of living bodies. In one aspect of the present invention, the mesenchymal stem cells are retained in the hydrogel and on the surface through a chemical bond, and can therefore be maintained without being eliminated from the hydrogel even when applied to an injured tendon tissue, thereby effectively promoting the regeneration of the tendon tissue.

Examples of the origin of the mesenchymal stem cells include bone marrow, fat, skeletal muscle, umbilical cord, dermis, and peripheral blood. Among them, mesenchymal stem cells derived from bone marrow are particularly preferably used. The origin of the cells can be autologous cells or allogeneic cells, and an undifferentiated state is preferred. A medium for use in the culture of the mesenchymal stem cells can be appropriately selected from known standard media depending on the type of the cells, etc. Specific examples thereof include Dulbecco's modified Eagle's medium (DMEM), NPBM, and $\alpha$-MEM. The medium can be further supplemented with serum such as FBS, a nutritional factor such as an amino acid, an antibiotic, a proliferation factor, a growth factor, and the like.

The mesenchymal stem cells play a role in serving as cross-linking points of the hydrogel by chemically binding to the water-soluble polymer, and therefore have a reactive group B capable of being covalently bonded to the reactive group A of the water-soluble polymer.

In the present specification, among the mesenchymal stem cells, cells can be used, for example, bone marrow mesenchymal stem cells derived from bone marrow and these bone marrow mesenchymal stem cells with high purity, dental pulp mesenchymal stem cells derived from dental pulp, or adipose mesenchymal stem cells derived from fat.

The type of the reactive group B can be appropriately set depending on the type of the reactive group A of the

water-soluble polymer and can be appropriately selected from, for example, an alkynyl group, an azide group, a carboxyl group, an amino group, a N-succinimide group, a N-hydroxysuccinimide ester group, a sulfhydryl group, a pyridyl disulfide group, and a maleimide group.

The reactive group B is preferably an alkynyl group or an azide group, more preferably an azide group, because the alkynyl group and the azide group can be chemically bonded conveniently by click chemistry (e.g., Huisgen reaction), as mentioned above.

For the existence of the reactive group B on the surface of the mesenchymal stem cells, for example, the reactive group B can be introduced to sialic acid contained in a sugar chain present on the surface of the cells. Specifically, cells having sialic acid bound with the reactive group B on the surface can be obtained by bonding the reactive group B to a sugar (e.g., mannosamine) metabolizable into sialic acid, and culturing the cells in a medium containing the sugar having the reactive group B.

More specifically, a bifunctional linker having a carboxyl group, a N-hydroxysuccinimide ester group, or a N-succinimide group and the reactive group B is used to bond the carboxyl group, the N-hydroxysuccinimide ester group, or the N-succinimide group of the bifunctional linker to an amino group of mannosamine and thereby synthesize mannosamine having the reactive group B. The cells are cultured in a medium containing the mannosamine having the reactive group B so that the mannosamine having the reactive group B is converted to sialic acid having the reactive group B through sugar metabolism reaction of the cells. This sialic acid having the reactive group B becomes a constituent of a sugar chain on the cell surface to obtain mesenchymal stem cells having the reactive group B on the surface. The mannosamine having the reactive group B for use in the culture of the cells may be allowed to lack a hydroxy group by acetylation or the like.

[Chem. 16]

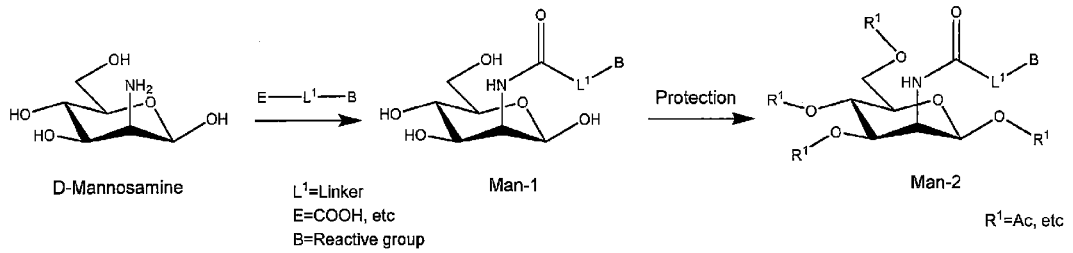


Such a lack of the hydroxy group moiety can place the reactive group B on sialic acid positioned at an end of a sugar chain on the cell surface, and facilitates forming a covalent bond to the water-soluble polymer. The concentration of the mannosamine having the reactive group B in the medium for the culture of the cells can be appropriately set depending on the amount of the reactive group B to be introduced, etc., and is, for example, 10 to 200 μM, preferably 10 to 100 μM, further preferably 40 to 100 μM. The culture time for the culture of the cells using the medium containing the mannosamine having the reactive group B is, for example, 6 to 96 hours, preferably 12 to 72 hours, further preferably 24 to 72 hours.

[Hydrogel]

In the biological material according to one aspect of the present invention, the water-soluble polymer having the reactive group A and the mesenchymal stem cells having the reactive group B capable of being covalently bonded to the reactive group A are allowed to coexist in an environment containing water so that the reactive group A and the reactive group B are covalently bonded to form a hydrogel with the cells as cross-linking points.

In one aspect, the content of the water-soluble polymer contained in the hydrogel to be formed can be appropriately set depending on the type of the water-soluble polymer used, etc. In the case of using, for example, alginic acid, the content thereof is, for example, approximately 0.1% by weight to approximately 20.0% by weight, approximately 0.2% by weight to approximately 8.0% by weight, or approximately 2.0% by weight to approximately 4.0% by weight, based on the total amount of the hydrogel.

In one aspect, the content of the cells contained in the hydrogel to be formed can be appropriately set depending on the site of a recipient tendon tissue, the severity of a disease, etc., and is, for example, approximately $1.0 \times 10^5$ cells/mL or more, approximately $5.0 \times 10^6$ to approximately $5.0 \times 10^7$, or approximately $1.0 \times 10^7$ to approximately $2.0 \times 10^8$ cells/mL.

In the present specification, the term "approximately" can include values up to ±10% of the numeric value in an aspect or up to ±20% of the numeric value in an aspect.

The hydrogel may further contain, optionally, a component promoting the proliferation of the cells, a medicinal component, a buffer, a stabilizer, and the like, in addition to the water-soluble polymer, the cells, and water.

For the formation of the hydrogel through the covalent bond between the reactive group A introduced in the water-soluble polymer and the reactive group B introduced in the cell surface, incubation can be performed, depending on the types of the reactive group A and the reactive group B, under conditions that attain the covalent bond therebetween. For example, when the combination of the reactive group A and the reactive group B is an alkynyl group and an azide group, the time required to form the hydrogel is, for example, several seconds to approximately 24 hours, several seconds to approximately 12 hours, several seconds to approximately 30 minutes, or several seconds to approximately 10 minutes.

The biological material according to one aspect of the present invention may be administered in a liquid or sol state so that the hydrogel is formed at an affected part. Particularly, the biological material in a liquid or sol state at the time of administration has fluidity and is thus capable of being administered with a syringe or the like. Therefore, an aspect in which the biological material is in a liquid or sol state at the time of administration and the hydrogel is formed at an affected part is preferred from the viewpoint of convenient handling, etc. For such a biological material in a liquid or sol state at the time of administration and the formation of the hydrogel at an affected part after administration, for example, reactive groups capable of being linked by click chemistry (e.g., an alkynyl group or an azide group) can be used as the reactive group A and the reactive group B. Since the formation of a covalent bond between the alkynyl group and the azide group progresses by the coexistence of these groups, the water-soluble polymer having the reactive group A and the cells having the reactive group B are mixed in an aqueous solvent such as water or saline before administration to an affected part to prepare a liquid or sol mixture. The liquid or sol mixture can be administered in an appropriate amount to the affected part with a syringe or the like before the formation of the covalent bond progresses to form a hydrogel. The administered liquid or sol mixture forms a hydrogel at the affected part as the formation of the covalent bond progresses by click chemistry.

The biological material according to another aspect of the present invention may form a hydrogel in advance, and the hydrogel itself can be administered to an affected part. The formation of the hydrogel in advance before administration can be performed by, for example, (1) a method of mixing the water-soluble polymer having the reactive group A and the cells having the reactive group B capable of being covalently bonded to the reactive group A in an aqueous solvent using a frame having a predetermined shape, and incubating them under conditions that attain the covalent bond between the reactive group A and the reactive group B to form a hydrogel having the desired shape, or (2) a method of mixing the water-soluble polymer having the reactive group A and the cells having the reactive group B capable of being covalently bonded to the reactive group A in an aqueous solvent, incubating them under conditions that attain the covalent bond between the reactive group A and the reactive group B to form a hydrogel, and then cutting the hydrogel into a predetermined shape.

When two or more hydrogels formed through the covalent bond between the reactive group A and the reactive group B using the water-soluble polymer having the reactive group A and the mesenchymal stem cells having the reactive group B are incubated in a contacted state of the hydrogels, one of the hydrogels is bound to the other hydrogel at contact surface by cell adhesion mediated by cadherin so that these two or more hydrogels can adhere to each other. Thus, in the case of forming a hydrogel in advance before administration, and administering the hydrogel itself to an affected part, two or more first hydrogels formed using the water-soluble polymer having the reactive group A and the mesenchymal stem cells having the reactive group B are prepared. Then, the two or more first hydrogels are further incubated in contact with each other so as to attain the desired shape to prepare a second hydrogel in which the two or more first hydrogels adhere to each other. This second hydrogel may be administered to an affected part. In this context, when the two or more first hydrogels are incubated in a contacted state, this incubation is performed by dipping in a medium that permits growth of the cells in a state where the two or more first hydrogels are contacted with each other. Since the two or more first hydrogels adhere to each other by cell-cell adhesion mediated by cadherin which requires a calcium ion, the medium desirably contains 0.2% by weight or more, preferably 0.2 to 1.0% by weight, of the calcium ion. Examples of the conditions for the incubation in a state where the two or more first hydrogels are contacted with each other include approximately 37°C for 6 to 24 hours.

[Usage of biological material]

The biological material provided herein is used for repairing a tendon tissue by administration to an injured site or a defective site of the tendon tissue. When the biological material of the present invention is applied to an injured or defective tendon tissue, the hydrogel fixed to an affected part is gradually hydrolyzed while the cells maintained in the hydrogel efficiently repair the tendon tissue. In the present specification, the "application" means that an affected part is loaded with the biological material in an amount sufficient for filling an injured part, a defective part, or the like of a tendon tissue. Examples of the subject to which the biological material is applied include humans and organisms other than humans, for example, birds and nonhuman mammals (e.g., bovines, monkeys, cats, mice, rats, guinea pigs, hamsters, pigs, dogs, rabbits, sheep, and horses).

In the present specification, the "tendon tissue" refers to a tissue constituted by tendon and muscle. The tendon is a band-like connective tissue that attaches bone to skeletal muscle. The tendon is composed mainly of type I collagen and contains a small amount of elastin and proteoglycan. The tendon also has a hierarchical structure of collagen fibrils, collagen fibers, and collagen fiber bundles, and the orientation of collagen fibers densely arranged in parallel with the major axis direction of the tendon is considered to influence the mechanical strength and functionality of the tendon. Examples of the tendon include tendon present at sites such as the upper extremities, the lower extremities, the head, the neck, the chest, the abdomen, the back, and the hip. More specific examples of the tendon include, but are not limited to, patella tendon, tibialis anterior tendon, Achilles tendon, popliteal tendon, semitendinosus tendon, gracilis tendon, abductor tendon, adductor tendon, supraspinatus tendon, infraspinatus tendon, subscapularis tendon, teres minor tendon, flexor tendon, rectus femoris tendon, posterior tibial tendon, and quadriceps femoris tendon.

In the present specification, the "injury of a tendon tissue" refers to a state where the tendon tissue is damaged due to aging, trauma, or other various factors, and includes a state having degeneration, inflammation, or the like in the tendon tissue, or a state with functional decline in the tissue. In the present invention, a site having injury in a tendon tissue is referred to as an "injured part of a tendon tissue". For example, a site having Achilles tendon injury is referred to as an "injured part of Achilles tendon". In some aspects of the present invention, the injured part of a tendon tissue occurs in at least a portion of the tendon tissue. In some aspects, the present invention relates to a biological material for Achilles tendon injury repair, the biological material repairing an injured part of Achilles tendon.

In the present specification, the "defect in a tendon tissue" refers to a part partially missing from an injured part of a tendon tissue, and refers to a hollow cavity of the tendon tissue and tissues surrounding the hollow cavity. In some aspects, the biological material is preferably used for repairing a "defective part of a tendon tissue", particularly, a "defective part of Achilles tendon". In the present specification, the "defect in a tendon tissue" is one form of "tendon tissue injury" and is encompassed by the term "tendon tissue injury". In the present specification, the "injury" also includes "rupture". The "rupture" includes rupture in various forms such as open rupture and subcutaneous rupture.

In the present specification, the "repair of a tendon tissue" does not require complete recovery of a tendon tissue after injury or defect into a state before the injury or the defect, and means improvement in the function of an injured or defective tendon tissue as compared with before application of the biological material. The "repair" can be used interchangeably with "treatment" or "regeneration". In one aspect of the present invention, the biological material is used for the treatment, prevention, or suppression of recurrence of tendon tissue injury. In the present specification, the "treatment, prevention, or suppression of recurrence" includes treatment, prevention, suppression of recurrence, regeneration, reduction, suppression, improvement, removal, decrease in incidence, delay of the time of onset, suppression of progression, mitigation of severity, decrease in recurrence rate, delay of the time of recurrence, alleviation of clinical symptoms, shortening of a rehabilitative period, and the like. In the present invention, the "prevention" includes not only prevention of the onset of a condition or a disease but delay of the time of onset and decrease in incidence. The "suppression of recurrence" includes not only complete suppression of the recurrence of a condition or a disease but delay of the time of recurrence and decrease in recurrence rate. In the present specification, the "treatment" is used in the meaning including the "treatment, prevention, or suppression of recurrence" described above, unless otherwise specified.

In one aspect of the present invention, the biological material can be applied to an affected part by, for example, transdermal administration or local administration through injection or the like. In another aspect, the biological material may be applied by indwelling in an incision made in an affected part. The number of times of application or the application frequency can be increased or decreased according to symptoms and effects. The biological material may be applied, for example, only once, or may be continuously administered every week or for several weeks. The application may be continued once a month or a year, irrespective of the period.

The dose of the biological material can be appropriately set depending on the site or state of a tendon tissue to be repaired, etc. Since the site given the biological material undergoes regeneration into a normal tissue, the dose of the biological material can be a dose so as to replenish a region to be regenerated.

In one aspect, the biological material of the present invention is in a gel or sol form when administered, and can be an injectable gel that transplants the cells in order to form a hydrogel at an affected part after administration.

[Production method]

The present invention also relates to a method for producing a biological material for tendon repair, comprising the step of subjecting a water-soluble polymer having a reactive group A and mesenchymal stem cells having a reactive group B to cross-linking reaction. In one aspect, the water-soluble polymer having the reactive group A is bAlg-DBCO, and the reactive group B is an azide group. More specifically, the production method according to one aspect of the present invention may comprise the steps of:

(1) preparing a solution containing the water-soluble polymer having the reactive group A;

(2) culturing mesenchymal stem cells in a medium containing a sugar having the reactive group B to introduce the reactive group B to the mesenchymal stem cells; and

(3) mixing the solution obtained in the step (1) with the mesenchymal stem cells obtained in the step (2) to obtain a hydrogel.

In the step (1), first, the reactive group A is introduced to a water-soluble polymer such as alginic acid. The method for introducing the reactive group A to the water-soluble polymer is as described in the section [Water-soluble polymer]. The water-soluble polymer having the reactive group A thus obtained is preferably converted into a branched compound using a branching agent. The solvent is not particularly limited as long as the solvent is applicable to a living body. The solvent is preferably an aqueous solvent, for example, purified water, pure water (e.g., distilled water or ion exchange water), ultrapure water (Milli-Q water), saline, phosphate-buffered saline, a HEPES buffer solution, or DMSO, preferably pure water or a HEPES buffer solution, more preferably a HEPES buffer solution. Such a solvent is preferably sterilized.

In the step (2), mesenchymal stem cells are cultured in a medium containing a sugar (e.g., mannosamine) bound with the reactive group B so that the reactive group B is introduced to sialic acid converted therefrom by sugar metabolism to introduce the reactive group B to the mesenchymal stem cells. The method for bonding the reactive group B to the sugar such as mannosamine, and the mesenchymal stem cells are as described in the section [Mesenchymal stem cell].

In the step (3), the solution obtained in the step (1) is mixed with the mesenchymal stem cells obtained in the step (2) to obtain a hydrogel. In this context, the "hydrogel" can be used interchangeably with a "cell cross-linked gel". The "cell cross-linked gel" means a gel that has formed a cell-water-soluble polymer macromolecular network structure with the cells themselves as cross-linking points of the water-soluble polymer.

The biological material of the present invention can be obtained by these steps.

[Treatment method]

The present invention also relates to a method for treating an injured part of a tendon tissue using the biological material mentioned above. More specifically, the present invention relates to the method comprising applying a hydrogel to an injured part of a tendon tissue in a subject in need of the treatment, the hydrogel comprising a water-soluble polymer having a reactive group A and mesenchymal stem cells having a reactive group B, and being obtained through cross-linking reaction of the reactive group A and the reactive group B.

An existing method for treating tendon tissue injury generally includes, for example, conservative therapy using suturation or a brace. However, such a treatment method might cause re-rupture without reaching repair into healthy tendon. Thus, there has been a demand for improvement in treatment method. Any existing product relating to a biological material for tendon repair has not yet been present. The treatment method using the biological material of the present invention is expected to shorten a treatment period, improve functional prognosis, shorten a rehabilitative period, and prevent re-division of tendon, for example, as compared with these existing treatment methods.

The treatment method according to one aspect of the present invention comprises steps given below. In each of the following steps, the term "optionally" means that the availability or degree of implementation of the step can be arbitrarily determined according to the state of a subject (test subject):

(a) optionally creating a visible state of an injured part of a tendon tissue by an incision, an arthroscope, or an endoscope;

(b) optionally removing unnecessary tissues in the injured part of the tendon tissue and a marginal region thereof;

(c) optionally suturing the injured part of the tendon tissue;

(d) applying the biological material of the present invention to the injured part of the tendon tissue; and

(e) optionally closing the incision or an opening made by the insertion, etc. of the arthroscope, the endoscope, or other instruments.

In the step (a), a visible state of an injured part of a tendon tissue is created by an incision, an arthroscope, or an endoscope before application of the biological material to the injured part of the tendon tissue. Local administration through injection or the like may not require creating a visible state of an injured part of a tendon tissue. The arthroscope is one type of endoscope for observing the state of joint.

In the step (b), at least a portion of the tendon tissue can be removed before application of the biological material to the injured part of the tendon tissue. The "unnecessary tissues in the injured part of the tendon tissue and a marginal region thereof are, for example, the injured part of the tendon tissue, a ruptured part, a degenerative part, a junction region between muscle and tendon, and abnormal tissue portions in their marginal regions.

The step (c) is the step of suturing the injured part of the tendon tissue before application of the biological

material. For example, in the case of large defect in Achilles tendon, the injured part is fixed by suture, and the biological material is then applied thereto. As a result, effective treatment is attained with the aim of functional recovery by suppressing further expansion of injury in Achilles tendon due to a load or exercise, and promoting tendon joining.

The step (d) is the step of applying the biological material to the injured part of the tendon tissue. The application of the biological material of the present invention is as described in the section [Usage of biological material], and the biological material can be applied to an affected part by, for example, transdermal administration, local administration through injection or the like, or indwelling in an incision. For the application of the biological material to the injured part of the tendon tissue, it is desirable to inject the biological material so as to sufficiently fill the cavity volume of an affected part. For example, in the case of applying the biological material in a liquid or sol state to Achilles tendon injury, it is desirable to inject the biological material until its liquid level becomes a height equivalent to tissues surrounding a defective part of Achilles tendon. In this case, as described in the section [Hydrogel], the biological material forms a hydrogel after application to an affected part, by the formation of a covalent bond between the reactive group A and the reactive group B by click chemistry. On the other hand, in the case of forming a hydrogel in advance before application to an affected part, and applying the hydrogel to the affected part, it is desirable to apply the biological material so as to sufficiently cover the affected part.

The step (e) is the step of subjecting the incision or an opening made by the insertion, etc. of the arthroscope, the endoscope, or other instruments to suture or a procedure equivalent thereto to close the opening. The term "other instruments" is not particularly limited as long as the instruments are general instruments that are usually used for operations. Examples thereof include instruments for arthroscopic surgeries, such as instruments for tissue excisions, instruments for sutures, and forceps.

A repair effect on the injured part of the tendon tissue using the biological material of the present invention can be determined by, for example, histological evaluation. Specifically, values converted to numeric values on the basis of a modified Watkins score and a modified Bonar scale can be used as indicators. Biomechanical evaluation such as a stretch test, or a gene expression test by PCR can also be used in combination therewith.

[Formulation and kit]

The present invention also relates to a kit for tendon repair, comprising: a water-soluble polymer having a reactive group A; and mesenchymal stem cells having a reactive group B, wherein the kit is used such that a hydrogel obtained through cross-linking reaction of the reactive group A and the reactive group B is applied.

The kit according to one aspect of the present invention comprises, for example, a glass bottle such as an ampule or a vial packed with a solution containing the water-soluble polymer having the reactive group A, and such a glass bottle packed with a solution containing the mesenchymal stem cells having the reactive group B. In another example, the kit comprises vials separately packed with the water-soluble polymer having the reactive group A and the mesenchymal stem cells having the reactive group B in their respective freeze-dried states. In this case, the kit desirably comprises a solvent (e.g., injectable water) that dissolves the freeze-dried products. In a further alternative example, the mesenchymal stem cells having the reactive group B may be in a cryopreserved state. The kit of the present invention can appropriately further comprise accessories such as a syringe, an injection needle, a dedicated filling machine, and an instruction manual.

A method for using the kit of the present invention can involve, for example, preparing a mixture of a solution containing the water-soluble polymer having the reactive group A and a solution containing the mesenchymal stem cells having the reactive group B before application to an affected part in a subject, and administering an appropriate amount of the resulting liquid or sol mixture to the affected part using a syringe or the like before the formation of the covalent bond progresses to form a hydrogel. The administered liquid or sol mixture is then applied as a hydrogel to the affected part as the covalent bond progresses. In another example, the kit may form a hydrogel in advance before administration, and the hydrogel can be applied to an affected part through an incision or the like. The method for forming the hydrogel before administration is as described in the section [Hydrogel].

[Examples]

Hereinafter, the present invention will be specifically described with reference to Examples. However, the present invention should not be construed by being limited by Examples given below. In Examples given below, One-way ANOVA was used in statistical study. A mean ± SD was calculated, and 0.05 or less was judged as being significant. GraphPad Prism (manufactured by GraphPad Software, Inc., v. 8) was used in analysis.

1. Synthesis of mannosamine in which azide group was introduced and hydroxy group was acetylated

Coupling reaction of D-mannosamine hydrochloride and azidoacetic acid was performed with DMT-MM (4-(4,6-dimethoxy-1,3,5-triazin-2-yl)-4-methylmorpholinium chloride) as a condensing agent. D-Mannosamine hydrochloride (200 mg, 0.93 mmol) was weighed and dissolved in 40 mL of ultrapure water (Milli-Q water). To the solution, DMT-MM (515 mg, 1.86 mmol) and azidoacetic acid (139 μL, 1.86 mmol) were added, and the mixture was heated to 45°C and reacted for 2 days. Two days later, the solvent was removed with an evaporator, and the residue was partially purified with methanol in order to remove precipitated by-products and unreacted matter. Column purification was performed by silica gel chromatography, and a solution was recovered only for a fraction that was able to be confirmed to contain the compound of interest by thin-layer chromatography (TLC). In this respect, the composition of the developing solvent used was methanol: chloroform = 2:1 (volume ratio). The synthesized compound was subjected to IR measurement to confirm that a compound with a group $-CO-CH_2-N_3$ bonded to an amino group of D-mannosamine (hereinafter, referred to as "ManNAz: N-azidoacetylmannosamine tetraacylated") was synthesized.

Next, four hydroxy groups of ManNAz were subjected to acetylation reaction. ManNAz and a flask as a reaction container were dried under reduced pressure for 1 day for dehydration. In a nitrogen atmosphere, ManNAz (200 mg, 0.76 mmol) was dissolved in pyridine (5.5 mL, 68.0 mmol). To the solution, acetic anhydride (0.6 mL, 6.84 mmol) was added, and the mixture was reacted for 30 minutes. The synthesized compound was purified by extraction using a funnel as described below. The compound was dissolved in dichloromethane, followed by extraction with a 1 M aqueous hydrochloric acid solution, a saturated aqueous solution of sodium bicarbonate, and saturated brine. The organic phase was dehydrated with magnesium sulfate and filtered by suction, and the solvent was removed from the recovered solution with an evaporator. The obtained synthesized compound was subjected to IR measurement, ESI-MS measurement, and $^1H$-NMR measurement to confirm that azidated mannosamine (hereinafter, referred to as "$Ac_4$ManNAz") was synthesized in which the group $-CO-CH_2-N_3$ was bonded to the amino group of D-mannosamine and four hydroxy groups of the D-mannosamine were acetylated.

2. Collection of bone marrow mesenchymal stem cell

All of operations were conducted in accordance with the regulations of an animal experiment committee at an animal experiment laboratory of Hokkaido University. Bone marrow mesenchymal stem cells (bone marrow stromal cells; BMSCs) were collected from LEW/SsNslc (male, 4 weeks old) by the method of Lennon et al. (Lennon DP. Isolation of rat marrow-derived mesenchymal stem cells. Exp Hematol. 2006; 34 (11): 1606-7). The rat was euthanized with sevoflurane and dipped in a beaker containing 70% ethanol for 3 minutes. The femur and the tibia were separated from hip joint, and muscle and ligaments were scraped off. The resulting bone was placed in a 100 mm dish containing PBS on ice. The bone was washed twice with PBS, and both ends of the bone were cut off, followed by the washing of bone tunnel with 10 mL of a culture solution (alpha-MEM, 20% FBS, 1% PenStrep, and 1% L-glutamine) using a syringe with an 18G needle. The washes were passed through a 70 μm cell strainer (manufactured by Greiner Bio-One International GmbH) and centrifuged at 450 G for 5 minutes. A fresh medium was added thereto, and the cells were transferred at a density of $9 \times 10^5$ cells/$cm^2$ to a culture vessel. Three days later, the medium was replaced with a fresh one to remove non-adherent cells. Medium replacement was performed at 2- to 3-day intervals, and the cells were subcultured using 0.25% trypsin when becoming 70% to 80% confluent. BMSCs of P2 were used in azidation, and BMSCs of P3 were used in cell transplantation.

3. Synthesis of branched alginate

Branched alginate (bAlg) was synthesized through coupling reaction of alginic acid sodium salt (manufactured by Sigma-Aldrich Co., LLC, Mw: 100,000) and 4-arm PEG-amine (manufactured by NOF Corp., SUNBRIGHT PTE-200PA, Mw: 20,000, CAS Registry No.: 804514-67-8) with DMT-MM as a condensing agent. Alginic acid sodium salt (100 mg, 1 μmol) was weighed and dissolved in 15 mL of ultrapure water (Milli-Q water). To the solution, DMT-MM (33.2 μg, 0.12 μmol) and 4-arm PEG-amine (2 mg, 0.1 μmol) were added to adjust the amount of the solution to a total of 20 mL. After 6 hours from the start of reaction, the solution was dialyzed for 2 days using a dialysis membrane (MWCO: 14,000) and further freeze-dried for 2 days to obtain a synthesized compound. As a result of examining its molecular composition by 1H-NMR measurement (D2O, 85°C), it was found that 0.1 molecules of 4-arm PEG were bound per molecule of alginic acid. This ratio was equivalent to alginic acid:4-arm-PEG-amine = 10: 1 (molar ratio) which was a mixing ratio in bAlg synthesis. It was thereby able to be confirmed that the bAlg of interest was able to be formed. The synthesis pathway of the branched alginate (bAlg) is shown below.

[Chem. 17]

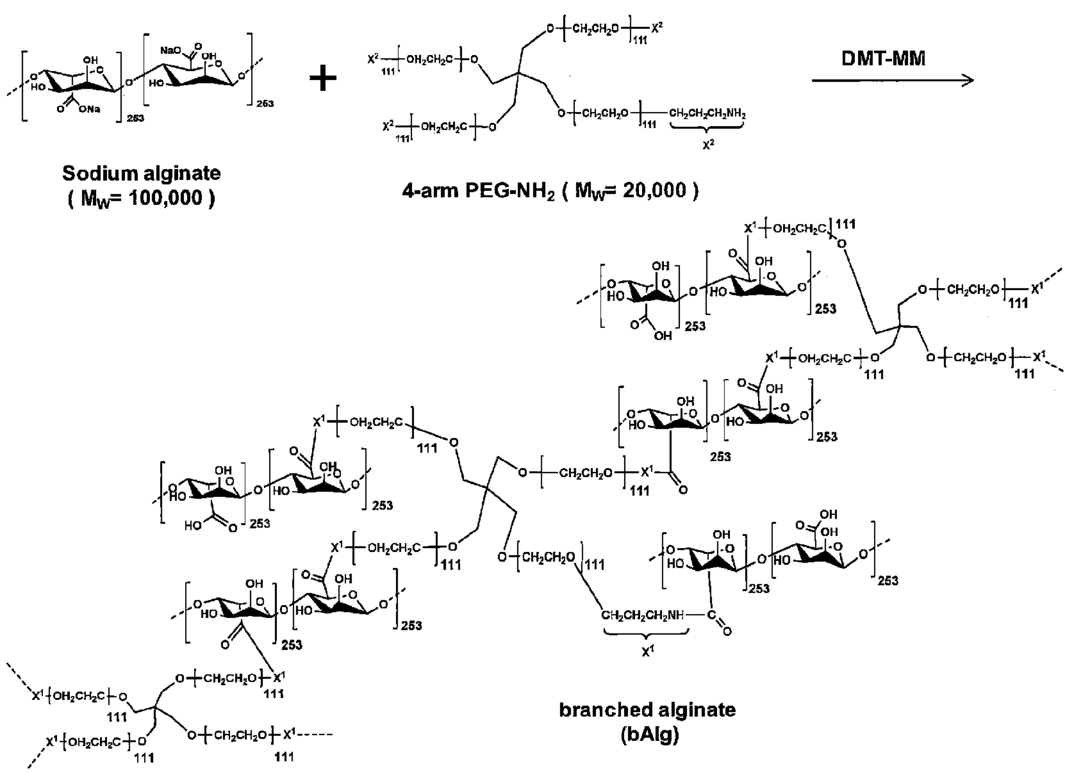


Sodium alginate
( $M_W$= 100,000 )

4-arm PEG-$NH_2$ ( $M_W$= 20,000 )

branched alginate
(bAlg)

In the formula (bAlg), $X^1$ = $-NHCH_2CH_2CH_2-$ is a binding pattern in which the imino group (-NH-) is bonded through an amide bond to a carboxyl group of alginate.

4. Synthesis of azadibenzocyclooctyne (DBCO)-modified branched alginate

bAlg (1000 mg, 1 μmol) was dissolved in 15 mL of ultrapure water (Milli-Q water). To the solution, DMT-MM (4.2 mg, 15.3 μmol) and DBCO-PEG4-amine (6.7 mg, 12.7 μmol; CAS Registry No.: 1255942-08-5) were added, and the mixture was shielded from light with aluminum foil and reacted at room temperature for 24 hours. Twenty-four hours later, the solution was dialyzed for 2 days using a dialysis membrane (MWCO: 14,000) and further freeze-dried for 2 days to obtain cyclooctyne-modified branched alginate (bAlg-DBCO). As a result of conducting 1H-NMR measurement ($D_2O$, 85°C), it was found that the number of DBCO molecules contained in bAlg-DBCO was 13. Thus, the approximate molecular weight of bAlg-DBCO was found to be 1,026,800 g/mol. The synthesis pathway of the cyclooctyne-modified branched alginate (bAlg-DBCO) is shown below.

[Chem. 18]

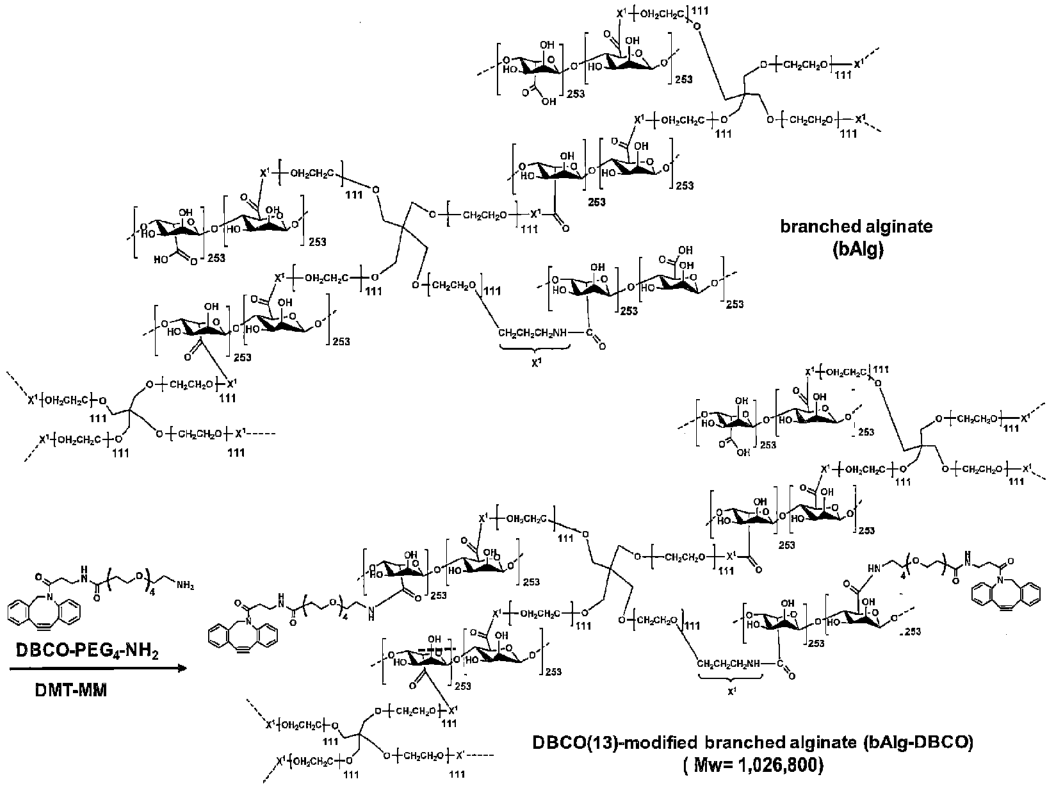


In the formula (bAlg-DBCO), $X^1$ = $-NHCH_2CH_2CH_2-$ is a binding pattern in which the imino group (-NH-) is bonded through an amide bond to a carboxyl group of alginate.

5. Preparation of cell cross-linked gel and cell-suspended gel

Rat bone marrow-derived mesenchymal stem cells (BMSCs) were inoculated at a density of $5 \times 10^5$ cells/T75 to a medium supplemented with $Ac_4ManNAz$ (100 μM), and cultured for 3 days for azidation. The cells were treated with 0.25% trypsin, then centrifuged, carefully pipetted, and centrifuged again. After complete removal of the medium, a 2% bAlg-DBCO solution (alginic acid manufactured by Sigma-Aldrich Co., LLC was used in bAlg; prepared with a HEPES buffer solution of pH 7.4) irradiated with ultraviolet ray for 1 hour was added to the azidated cells such that the density was $2 \times 10^6$ cells/mL to prepare a cell cross-linked gel. Likewise, cell suspensions were prepared from BMSCs at a density of $2 \times 10^6$ cells/mL using 2% ultra purified alginic acid (UPAL; UPAL500 manufactured by MOCHIDA PHARMACEUTICAL CO., LTD..., Lot No. 5H15) and 2% alginic acid (AL; A2033 manufactured by Sigma-Aldrich Co., LLC) irradiated with ultraviolet ray for 1 hour. Figure 1 shows a schematic view of the process of preparing the cell cross-linked gel.

6. Defect in rat Achilles tendon and transplantation

LEW/SsNslc (male, 8 weeks old) was intraperitoneally anesthetized (75 mg/kg ketamine and 0.5 mg/kg medetomidine) and shaved, and a medial vertical skin incision of approximately 10 mm was then made. Defect in Achilles tendon and transplantation were performed by the method of Aspenberg et al. (Aspenberg P. Platelet concentrate injection improves Achilles tendon repair in rats. Acta Orthop Scand. 2004; 75 (1): 93-9). 3 mm defect was made in a central part of Achilles tendon with a sharp-pointed knife to also cause defect in plantar tendon (Figure 2). The epidermis was sutured with 8-0 Nylon without suturing the defective part of Achilles tendon. Individual rats were randomly assigned to six treatment groups.

- Saline administration (control group)
- Administration of AL (manufactured by Sigma-Aldrich Co., LLC) or UPAL 500 (manufactured by MOCHIDA PHARMACEUTICAL CO., LTD..) alone (AL group or UPAL group)

- BMSCs-suspended AL (manufactured by Sigma-Aldrich Co., LLC) or UPAL 500 (manufactured by MOCHIDA PHARMACEUTICAL CO., LTD..) gel (MSC-A group or MSC-U group)
- Cell cross-linked gel using AL-DBCO 10 (manufactured by Sigma-Aldrich Co., LLC) (cross-link-A group)

After wound closure, 50 μL of each solution for transplantation was transdermally injected thereto with a 25G needle. Neither postoperative fixation nor non-weight bearing was performed. The rats were euthanized on postoperative weeks 2 and 4 and subjected to histological evaluation, qPCR, and a stretch test. The left and right sides of the rats for the stretch test were used as healthy and affected sides, respectively.

As a result of megascopic evaluation, no postoperative infection was observed at any point in time. In all the groups, the defective part of Achilles tendon was found to have continuity from an early stage on postoperative week 2 (regions indicated by the arrows of Figure 3 are defective portions having continuity). In all the treatment groups, the proliferation of blood vessels was observed in the superficial part of Achilles tendon. On postoperative week 4, the margin of Achilles tendon was defined, and the proliferation of superficial blood vessels disappeared. No megascopic difference was observed in any of the groups.

7. Histological evaluation

Distal 1/3 of triceps surae muscle to 1/2 of calcaneus was excised to obtain Achilles tendon. The tendon was fixed in 10% formalin for 1 day and decalcified with EDTA for 4 weeks to prepare a paraffin block. 5 μm slices were prepared such that the cut surface was located at the center of the sagittal plane of Achilles tendon, followed by deparaffinization. Then, the slices were stained with HE and stained with an anti-collagen type I antibody (manufactured by Sigma-Aldrich Co., LLC; C2456) and an anti-collagen type III antibody (manufactured by Thermo Fisher Scientific Inc.; MA1-22147). The slices were observed under an optical microscope BZ-X710 (manufactured by Keyence Corp.) and a polarizing microscope (manufactured by Nikon Corp., ECLIPSE-E600-POL). For the quantitative evaluation of repaired tissues in each treatment group, histological findings were converted to numeric values with 24 points at the maximum using modified Watkins scores (Table 4) of Ide et al. (Ide J. The effects of fibroblast growth factor-2 on rotator cuff reconstruction with acellular dermal matrix grafts. Arthroscopy. 2009; 25 6): 608-16). Findings obtained under a phase shift laser microscope (PLM) were converted to numeric values on the basis of partial excerpts (Table 5) from a modified Bonar scale (Loppini M. Histopathological scores for tissue-engineered, repaired and degenerated tendon: a systematic review of the literature. Curr Stem Cell Res Ther. 2015; 10 (1): 43-55).

[Table 4]

| | 1 | 2 | 3 | 4 |
|---|---|---|---|---|
| Cellularity | Marked | Moderate | Mild | Minimal |
| Proportion of cells resembling tenocytes | <25% | 25-50% | 50-75% | >75% |
| Proportion of cells oriented parallel | <25% | 25-50% | 50-75% | >75% |
| Vascularity | Marked | Moderate | Mild | Minimal |
| Proportion of fibers with large diameter | <25% | 25-50% | 50-75% | >75% |
| Proportion of fibers oriented parallel | <25% | 25-50% | 50-75% | >75% |

[Table 5]

| | 1 | 2 | 3 | 4 |
|---|---|---|---|---|
| Crimping collagen formation/fiber structure | Having no fiber structure | Fibers with ill-defined boundary | Fibers with defined boundary | Dense fibers Smooth fibers Uniform polarized nature |

Six groups, the control group, the UPAL group (UPAL 500 alone), the MSC-U group (UPAL 500 + BMSCs), the AL group (AL (manufactured by Sigma-Aldrich Co., LLC) alone), the MSC-A group (AL (manufactured by Sigma-Aldrich Co., LLC) + BMSCs), and the cross-link-A group (cell cross-linked gel using AL-DBCO 10), were evaluated at points in time of postoperative weeks 2 and 4 (each N = 4). On postoperative week 2, all the groups had a high cell density, spindle-shaped tendon cells few in number, and poor orientation of the cells (Figure 4). Also, the proliferation of blood vessels was observed. Collagen fibers had poor continuity and orientation and had a small diameter. Continuous

fibers were not observed in PLM, whereas fibers having partial continuity were observed in the cross-link-A group which tended to have a large PLM score (Figure 5). The modified Watkins scores on postoperative week 2 were the control group (9.33 ± 0.94), the UPAL group (9.00 ± 2.35), the MSC-U group (9.50 ± 1.50), the AL group (9.00 ± 0), the MSC-A group (9.33 ± 0.47), and the cross-link-A group (10.5 ± 1.12) with no significant difference found among these six groups (Figure 6). On postoperative week 4, the cell density was decreased, and the number of spindle-shaped tendon cells was increased (Figure 7). The cross-link-A group compared with the AL group exhibited significant cell orientation in a parallel direction (P = 0.0430). The proliferation of blood vessels tended to be decreased and was significantly decreased in the UPAL group (P = 0.0205, 0.0205), the MSC-U group (P = 0.0205, 0.0205), and the cross-link-A group (P = 0.0205, 0.0205) compared with the AL group and the MSC-A group. Collagen fibers, albeit having a small diameter, exhibited continuity in all the groups and tended to have strong stainability. The cross-link-A group compared with the control group, the AL group, and the MSC-A group had significant orientation of collagen fibers in a parallel direction (P = 0.0255, 0.0082, 0.0082). In PLM, continuous fibers were observed in all the groups, and the cross-link-A group had many collagen fiber bundles and tended to have a large score (Figure 8). The modified Watkins scores were the control group (11.67 ± 0.94), the UPAL group (14.00 ± 0.71), the MSC-U group (14.00 ± 0.71), the AL group (11.67 ± 0.94), and the MSC-A group (10.50 ± 1.80), and the score of the cross-link-A group (15.00 ± 1.22) was significantly higher than the values of the control group, the AL group, and the MSC-A group (P = 0.0407, 0.0019, 0.0056) (Figure 9). As for sub-entry, the items of cell orientation (control group; 1.33 ± 0.47, cross-link-A group; 2.00 ± 0.00) and fiber orientation (control group; 1.33 ± 0.47, cross-link-A group; 2.50 ± 0.50) were significantly higher values in the cross-link-A group than in the control group ($p < 0.05$).

8. qPCR

A central part of tendon was harvested, and RNA was extracted in accordance with the protocol of RNeasy Fibrous Tissue Kit (manufactured by Qiagen N.V.). cDNA was synthesized from 1.0 μg of the RNA of each sample using random hexamer primers (manufactured by Promega Corp.) and ImProm II reverse transcriptase (manufactured by Promega Corp.). Gene expression was measured in Thermal Cycler Dice Real Time System II (manufactured by Takara Bio Inc.) using 2 ng of each cDNA, SYBR Premix Ex Taq II (manufactured by Takara Bio Inc.), and gene-specific primers. qPCR was performed in triplicate. For mRNA expression, the expression level of each mRNA was normalized with a Ct value of GAPDH by use of the ΔΔCt method and further normalized with a mean expression level of each mRNA in healthy Achilles tendon samples.

Four groups, the control group, the UPAL group (UPAL 500 alone), the MSC-U group (UPAL 500 + BMSCs), and the cross-link-A group, were examined on postoperative week 2 (N = 3) (Figure 10). Gene expression was examined as to mature collagen Col1, immature tendon collagen Col3, Scleraxis (Scx) related to tendon maturation, Tenascin C (Tnc) expressed at the stage of tendon development, type II transmembrane glycoprotein Tenomodulin (Tnmd) relatively specific for tendon cells, proteoglycan Decorin (Dcn) constituting tendon, and Smad9 related to tendon cell differentiation of mesenchymal stem cells.

9. Stretch test

Tendon was biomechanically evaluated using a stretch tester (manufactured by Shimadzu Corp., Tensile Tester AG-X 20kN). Before the stretch test, the thickness and width of the tendon were measured in CL-3000 series multicolor laser coaxial displacement meter (manufactured by Keyence Corp., optical unit model No.: CL-L015 (2-head specification)). Triceps surae muscle and calcaneus were attached to the stretch tester using a fixation with sandpaper. Tendon was pulled in a vertical direction at a rate of 10 mm/min until completely broken. Ultimate load and stiffness were calculated using TRAPEZIUM X (manufactured by Shimadzu Corp., Ver. 1.2.2). For comparison with the treatment groups, contralateral sides were similarly measured as healthy tendon.

Six groups, the control group, the AL group, the MSC-A group, the UPAL group, the MSC-U group, and the cross-link-A group, were subjected to the stretch test on postoperative week 4 (each N = 6) (Figure 11). In each group, the contralateral left side was similarly subjected to the stretch test without any operation, and used to normalize a numeric value on the affected side. The ultimate load of the control group, the AL group, the MSC-A group, the UPAL group, the MSC-U group, and the cross-link-A group was 39.04 ± 7.18, 32.77 ± 3.91, 38.78 ± 4.60, 31.85 ± 4.42, 36.63 ± 9.46, and 43.92 ± 5.93, respectively, with no significant difference. These values were normalized on the healthy side into 0.87 ± 0.15, 0.88 ± 0.16, 0.87 ± 0.19, 0.81 ± 0.14, 0.89 ± 0.20, and 1.18 ± 0.29 N, respectively, and tended to be high in the cross-link-A group. The ultimate load/cross-sectional area of the control group, the AL group, the MSC-A group, the UPAL group, the MSC-U group, and the cross-link-A group was 4.46 ± 1.14, 4.66 ± 0.83, 4.48 ± 0.85, 2.84 ± 0.43, 3.83 ± 1.16, and 5.67 ± 1.21 N/mm2, respectively. The cross-link-A group compared with the AL group had a significantly high value (P = 0.0011). These values were normalized on the healthy side into 0.21 ± 0.04, 0.251 ± 0.08, 0.221 ± 0.06, 0.151 ± 0.03, 0.251 ± 0.08, and 0.341 ± 0.07, respectively, and exhibited no significance.

The stiffness of the control group, the AL group, the MSC-A group, the UPAL group, the MSC-U group, and the cross-link-A group was 12.96 ± 1.12, 11.47 ± 2.41, 15.41 ± 4.36, 12.54 ± 1.62, 11.59 ± 2.51, and 18.75 ± 4.00 N/mm, respectively. The cross-link-A group compared with the control group, the AL group, and the MSC-A group had a significantly high value (P = 0.0427, 0.0251, 0.0071). These values were normalized on the healthy side into 0.37 ± 0.08, 0.34 ± 0.10, 0.47 ± 0.15, 0.36 ± 0.03, 0.44 ± 0.21, and 0.75 ± 0.43, respectively. The cross-link-A group compared with the control group and the AL group had a significantly high value (P = 0.0252, 0.0007).

**Claims**

1. A biological material for tendon repair, comprising a hydrogel formed through cross-linking reaction of a reactive group A and a reactive group B, wherein
the hydrogel comprises:

    a water-soluble polymer having the reactive group A; and
    mesenchymal stem cells having the reactive group B.

2. The material according to claim 1, wherein a combination of the reactive group A and the reactive group B is selected from the group consisting of combinations in the following table:

[Table 6]

| | Reactive group A | Reactive group B |
|---|---|---|
| RG-1 | Alkynyl group | Azide group |
| RG-2 | Azide group | Alkynyl group |
| RG-3 | Carboxyl group | Amino group |
| RG-4 | N-Hydroxysuccinimide ester group | Amino group |
| RG-5 | N-Succinimide group | Amino group |
| RG-6 | Amino group | Carboxyl group |
| RG-7 | Amino group | N-Hydroxysuccinimide ester group |
| RG-8 | Amino group | N-Succinimide group |
| RG-9 | Sulfhydryl group | Sulfhydryl group |
| RG-10 | Sulfhydryl group | Pyridyl disulfide group |
| RG-11 | Sulfhydryl group | Maleimide group |
| RG-12 | Pyridyl disulfide group | Sulfhydryl group |
| RG-13 | Maleimide group | Sulfhydryl group |

3. The material according to claim 1, wherein the reactive group A is an alkynyl group, and the reactive group B is an azide group.

4. The material according to claim 1, wherein the reactive group A is a cyclic alkyne group, and the reactive group B is an azide group.

5. The material according to claim 1, wherein the water-soluble polymer comprises alginic acid bound with a branched compound.

6. The material according to claim 1, wherein the water-soluble polymer comprises alginic acid bound with a branched compound selected from the group consisting of the following formulas (1-N), (1-N-1), (1-N-2-1), and (1-N-2-2):

[Table 7]

| | |
|---|---|
| Formula (1-N) | 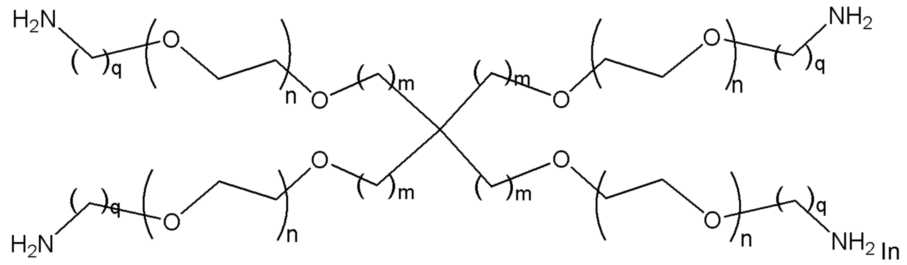<br>formula (1-N), m = 1-10, n = 10-500, q = 1-10 |
| Formula (1-N-1) | 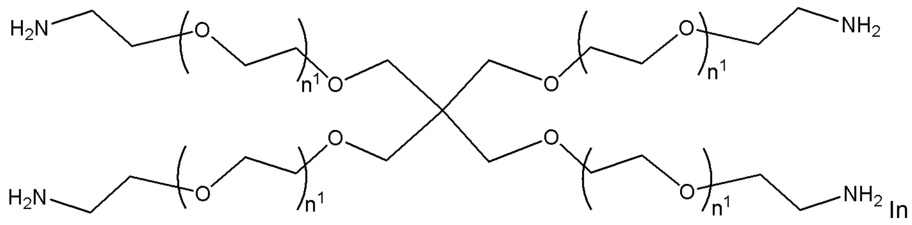<br>formula (1-N-1), $n^1$ = 10-500 |
| Formula (1-N-2-1) | 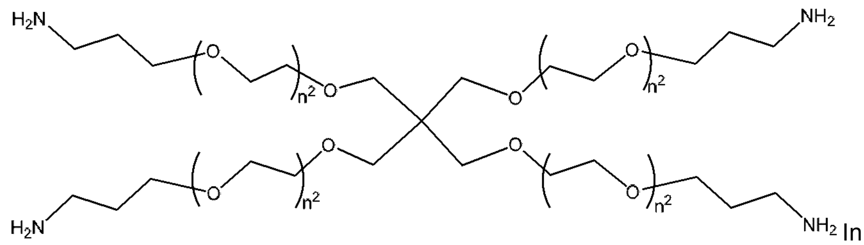<br>formula (1-N-2-1), $n^2$ = 10-500 |
| Formula (1-N-2-2) | 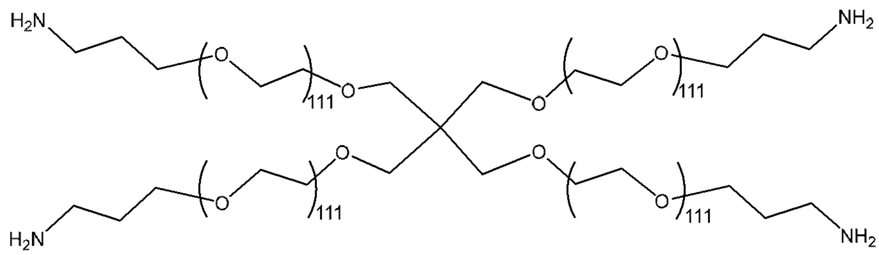 |

**7.** The material according to claim 1, wherein the water-soluble polymer comprises branched alginate (bAlg) represented by the following formula:

[Chem. 19]

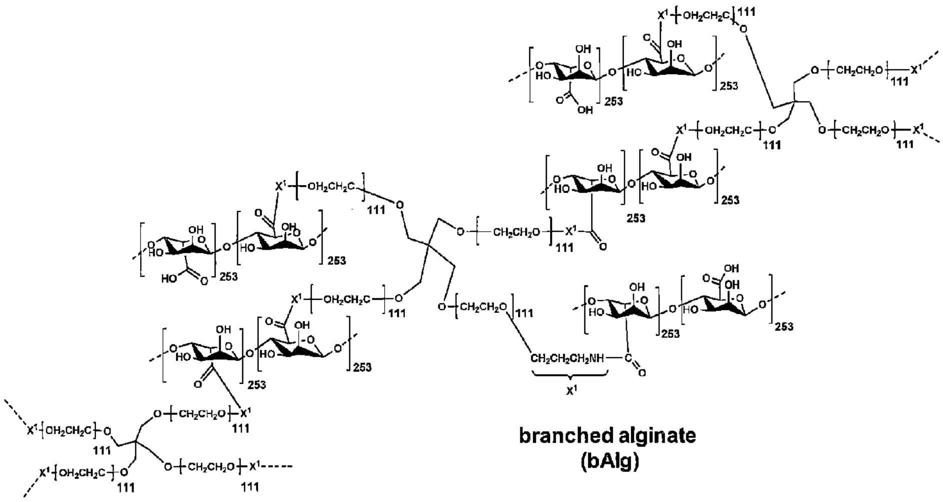

branched alginate (bAlg)

wherein $X^1$ = $-NHCH_2CH_2CH_2-$ is a binding pattern in which the imino group is bonded through an amide bond to a carboxyl group of alginate.

**8.** The material according to claim 1, wherein the water-soluble polymer having the reactive group A is cyclooctyne-modified branched alginate (bAlg-DBCO) represented by the following formula:

[Chem. 20]

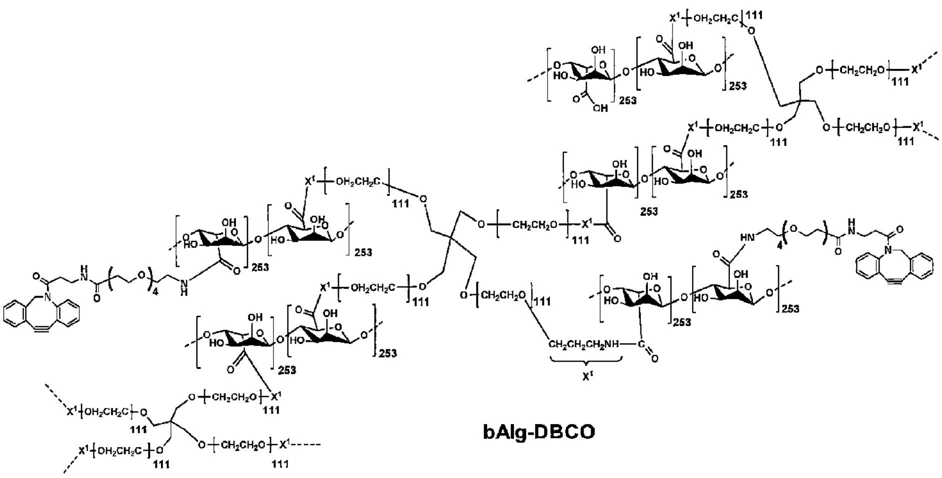

bAlg-DBCO

wherein $X^1$ = $-NHCH_2CH_2CH_2-$ is a binding pattern in which the imino group is bonded through an amide bond to a carboxyl group of alginate.

**9.** The material according to claim 1, wherein the mesenchymal stem cells are derived from bone marrow.

**10.** The material according to claim 1, wherein the number of cells contained in the hydrogel is $1.0 \times 10^5$ cells/mL or more.

**11.** The material according to claim 1, wherein a concentration of the water-soluble polymer is 0.2% by weight to 8.0% by weight based on the total amount of the hydrogel.

**12.** The material according to claim 1, wherein the hydrogel is an injectable gel.

**13.** The material according to any one of claims 1 to 12, wherein the material is used for the repair of a tendon tissue selected from the group consisting of patella tendon, tibialis anterior tendon, Achilles tendon, popliteus tendon, semitendinosus tendon, gracilis tendon, supraspinatus tendon, infraspinatus tendon, subscapularis tendon, teres minor tendon, rectus femoris tendon, posterior tibial tendon, and quadriceps femoris tendon.

**14.** A method for producing a biological material for tendon repair, comprising the step of subjecting a water-soluble polymer having a reactive group A and mesenchymal stem cells having a reactive group B to cross-linking reaction.

**15.** The production method according to claim 14, wherein the water-soluble polymer having the reactive group A is cyclooctyne-modified branched alginate (bAlg-DBCO), and the reactive group B is an azide group.

**16.** A kit for tendon repair, comprising:

a water-soluble polymer having a reactive group A; and
mesenchymal stem cells having a reactive group B, wherein
the kit is used such that a hydrogel obtained through cross-linking reaction of the reactive group A and the reactive group B is applied.

**17.** A composition for use in tendon repair comprising a water-soluble polymer having a reactive group A.

**18.** A composition for use in tendon repair comprising a water-soluble polymer having a reactive group A and mesenchymal stem cells having a reactive group B.

**19.** A composition for use in tendon repair, comprising a hydrogel formed through cross-linking reaction of a water-soluble polymer having a reactive group A and mesenchymal stem cells having a reactive group B.

FIG. 1

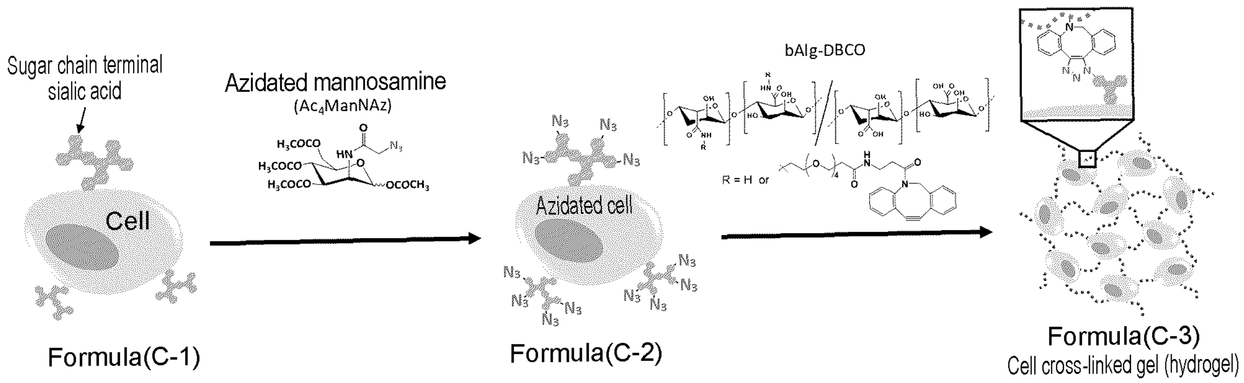
Sugar chain terminal sialic acid
Cell
Azidated mannosamine
(Ac4ManNAz)
Azidated cell
bAlg-DBCO
Formula(C-1)
Formula(C-2)
Formula(C-3)
Cell cross-linked gel (hydrogel)

FIG. 2

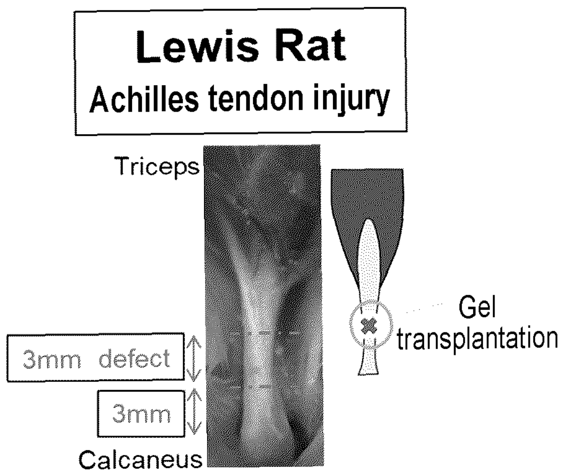
Lewis Rat
Achilles tendon injury
Triceps
3mm defect
3mm
Calcaneus
Gel
transplantation

FIG. 3

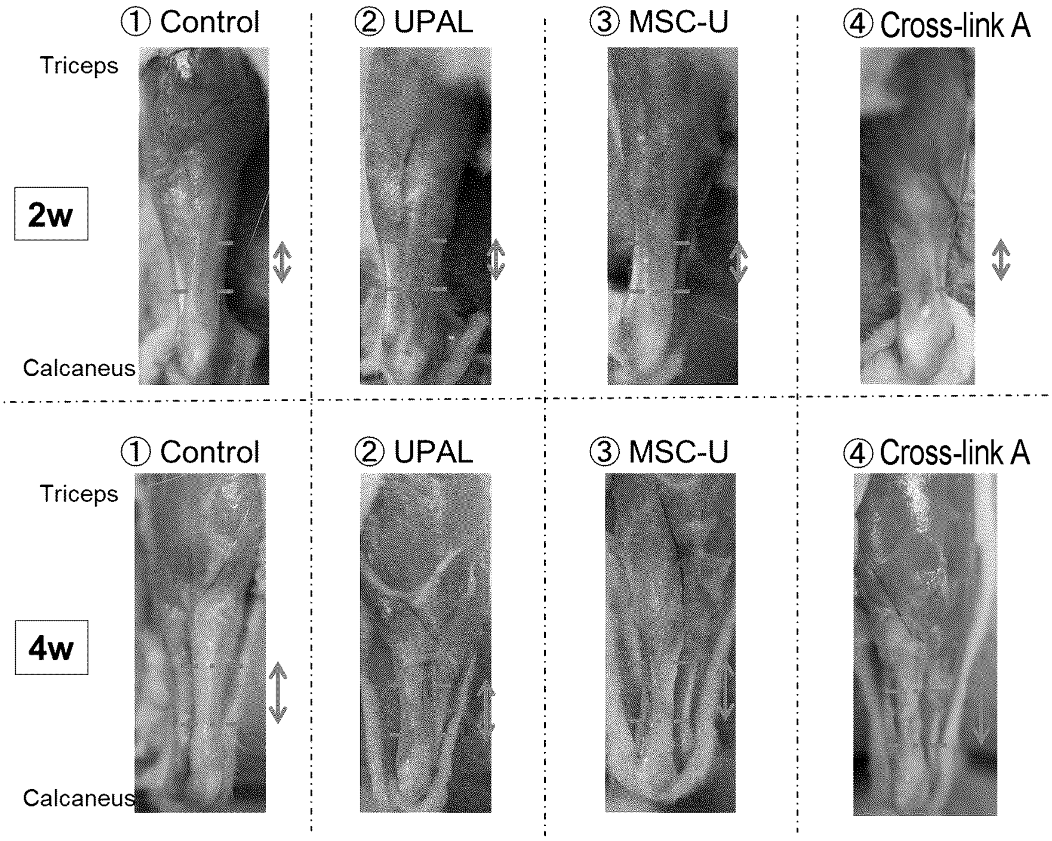
① Control
② UPAL
③ MSC-U
④ Cross-link A
Triceps
2w
Calcaneus
① Control
② UPAL
③ MSC-U
④ Cross-link A
Triceps
4w
Calcaneus

FIG. 4

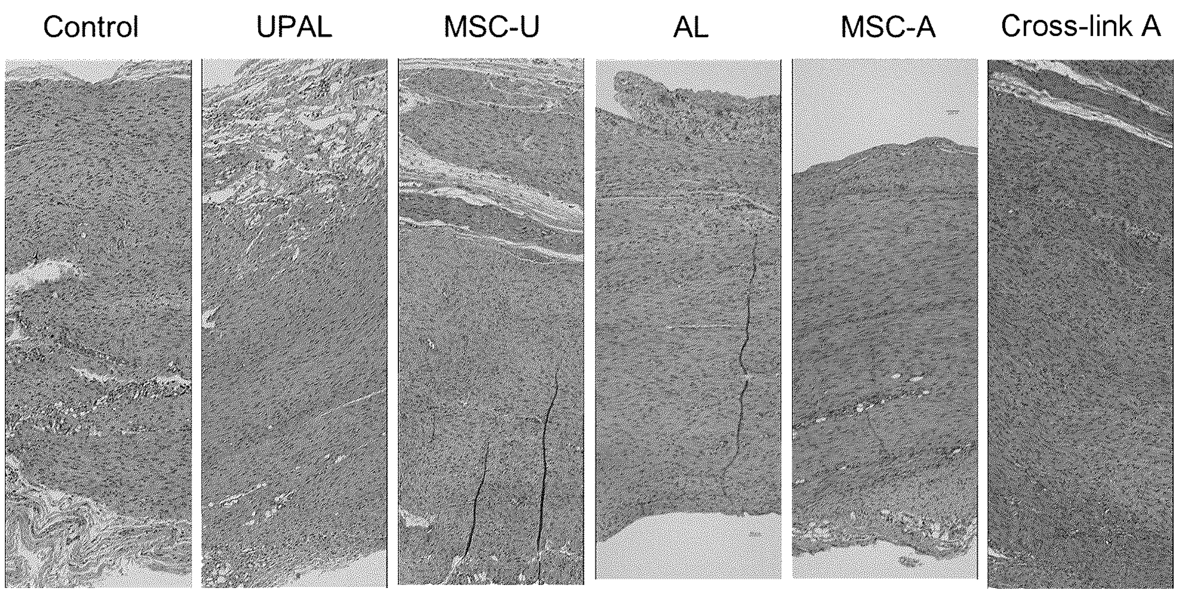
Control
UPAL
MSC-U
AL
MSC-A
Cross-link A

FIG. 5

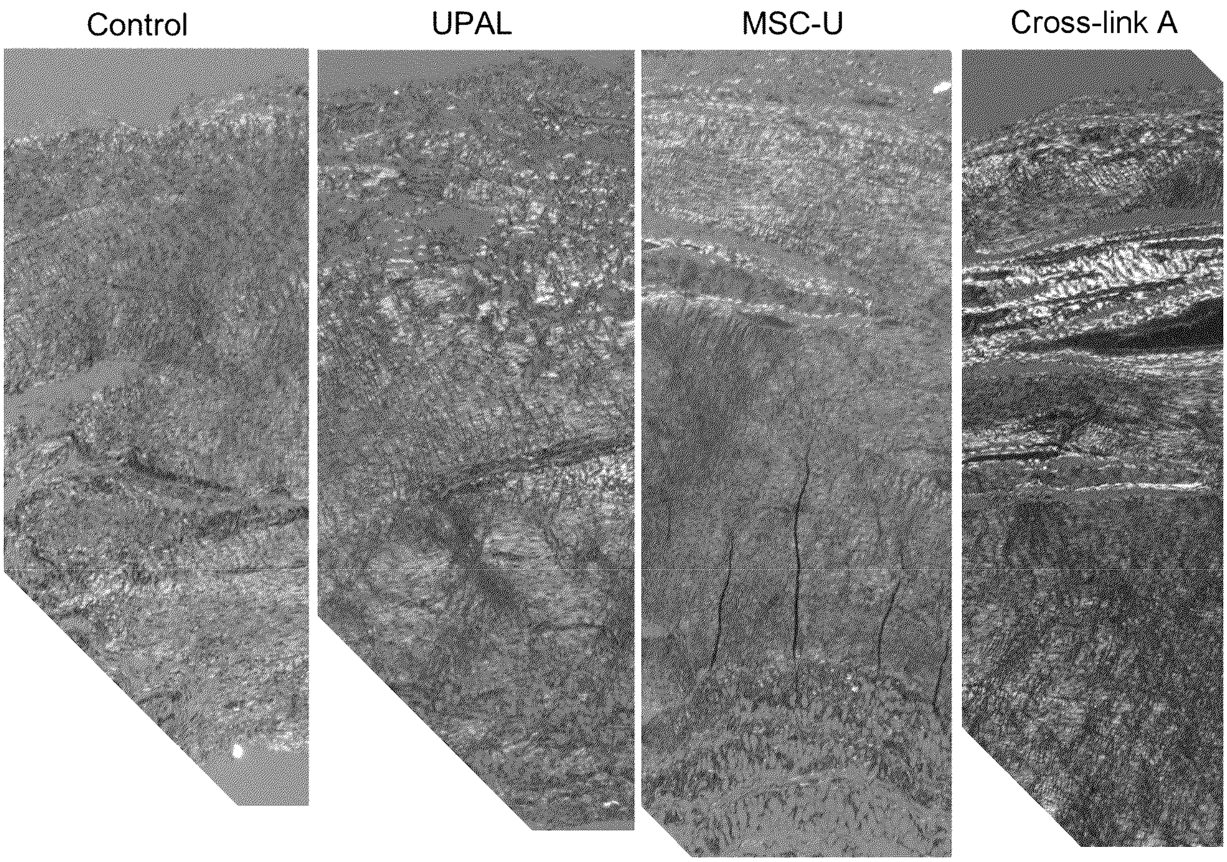
Control
UPAL
MSC-U
Cross-link A

FIG. 6

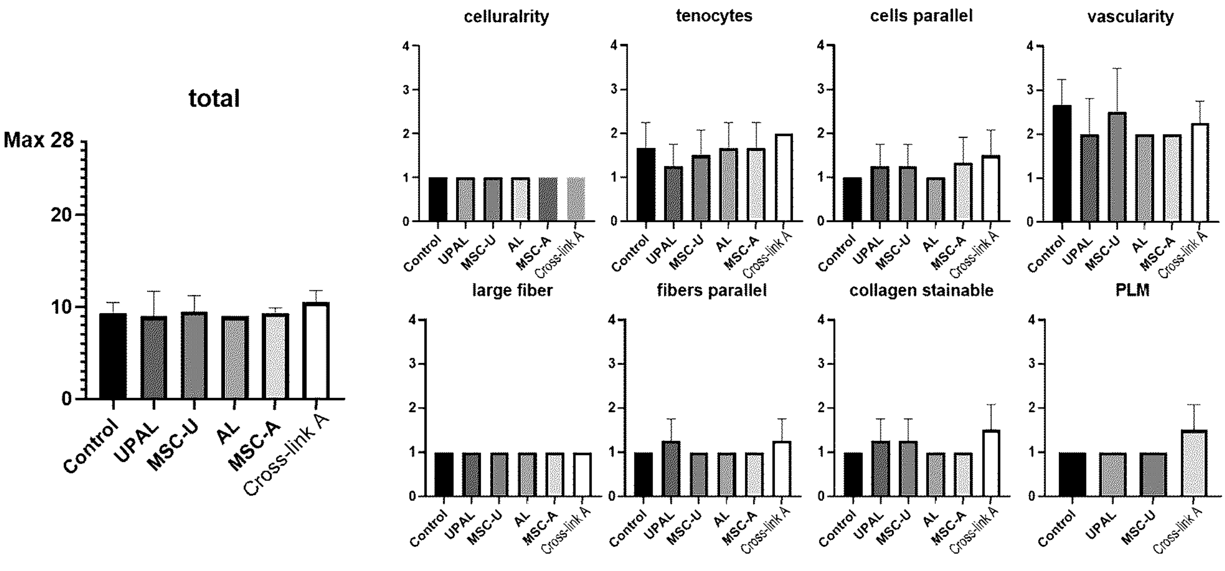
total
Max 28
20
10
0
Control
UPAL
MSC-U
AL
MSC-A
Cross-link A
celluralrity
tenocytes
cells parallel
vascularity
large fiber
fibers parallel
collagen stainable
PLM

FIG. 7

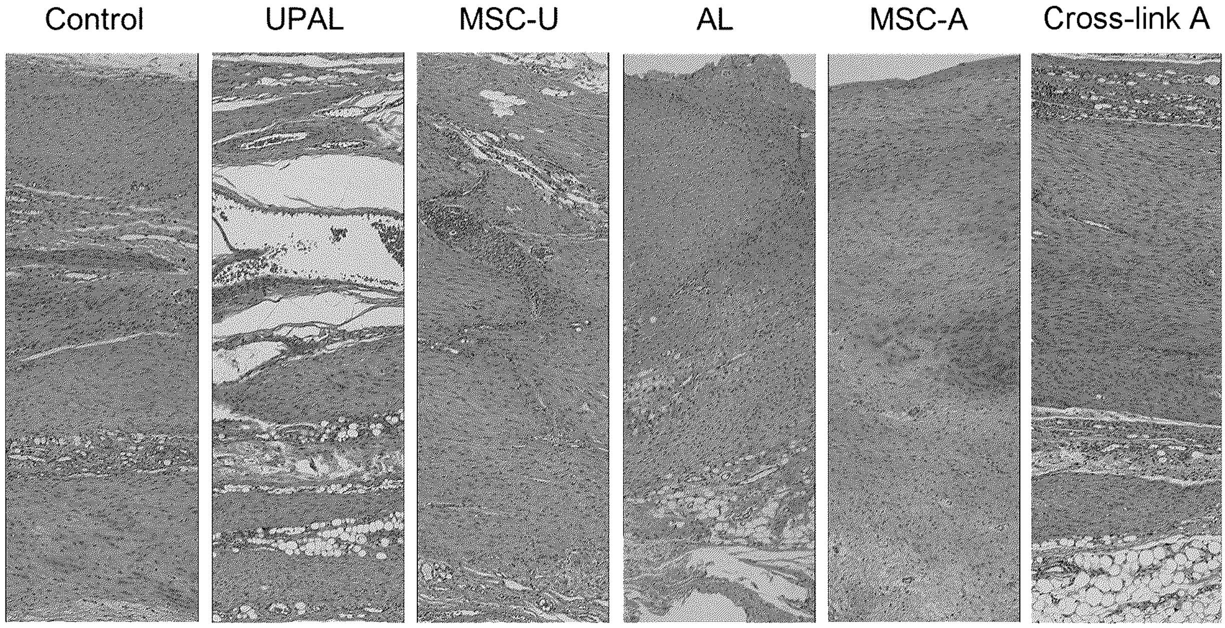
Control
UPAL
MSC-U
AL
MSC-A
Cross-link A

FIG. 8

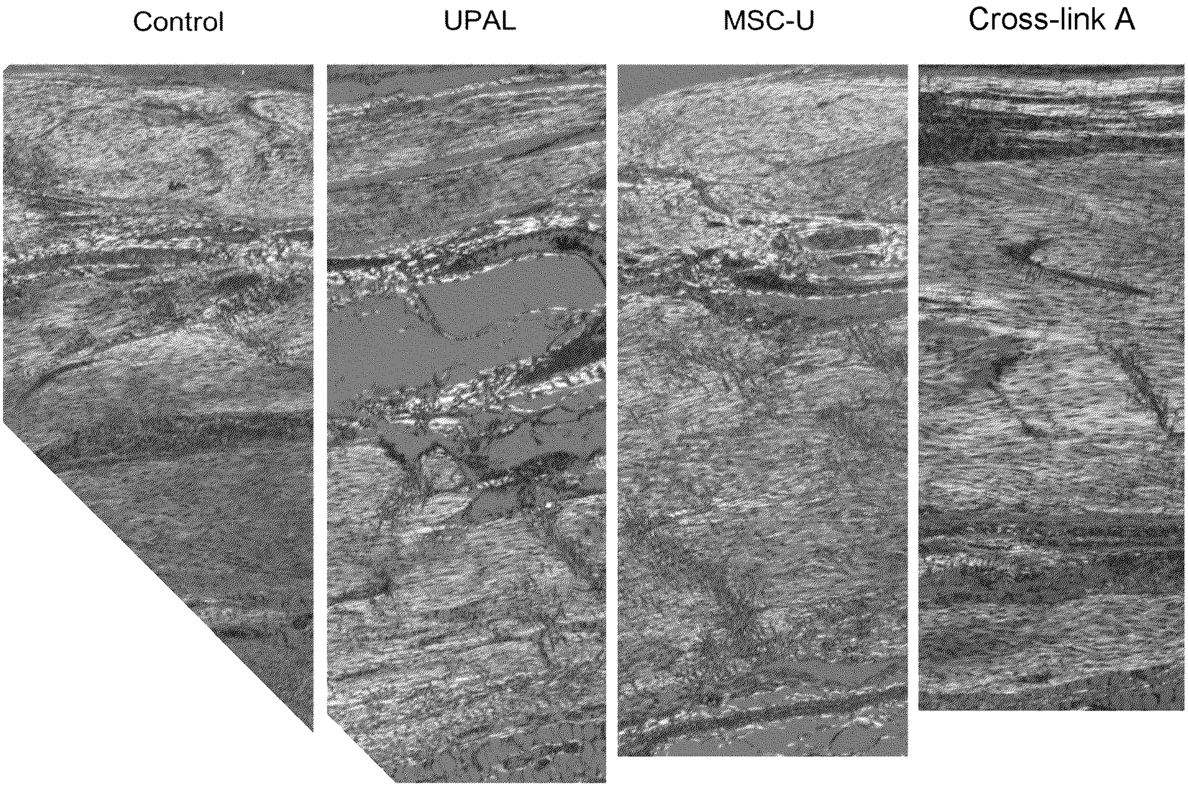
Control
UPAL
MSC-U
Cross-link A

FIG. 9

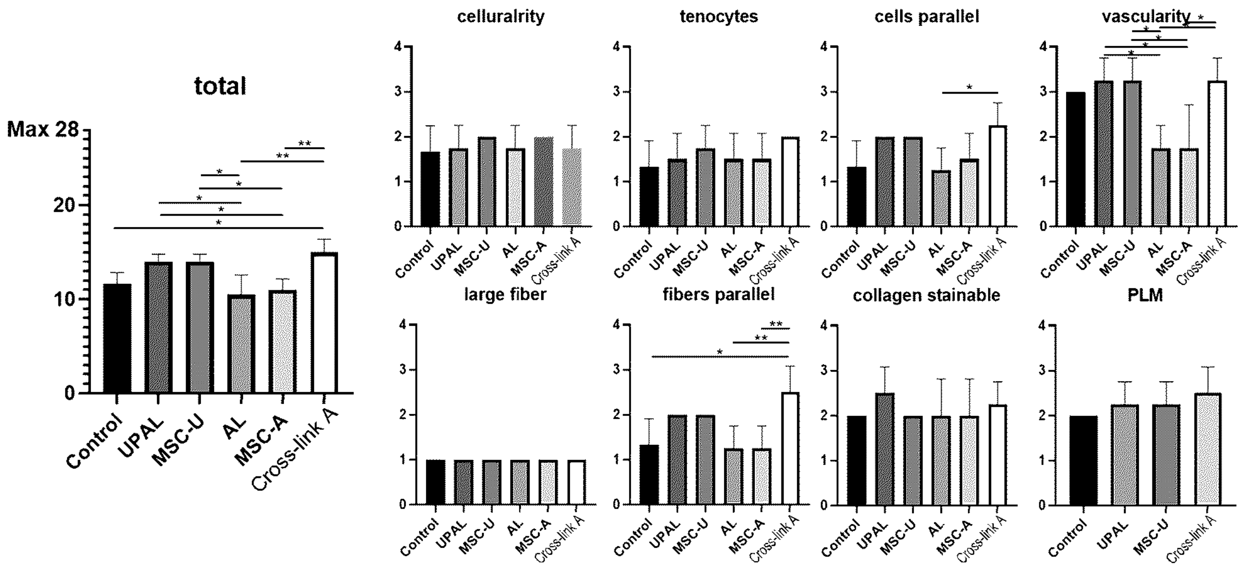
total
Max 28
celluralrity
tenocytes
cells parallel
vascularity
large fiber
fibers parallel
collagen stainable
PLM
Control
UPAL
MSC-U
AL
MSC-A
Cross-link A

FIG. 10

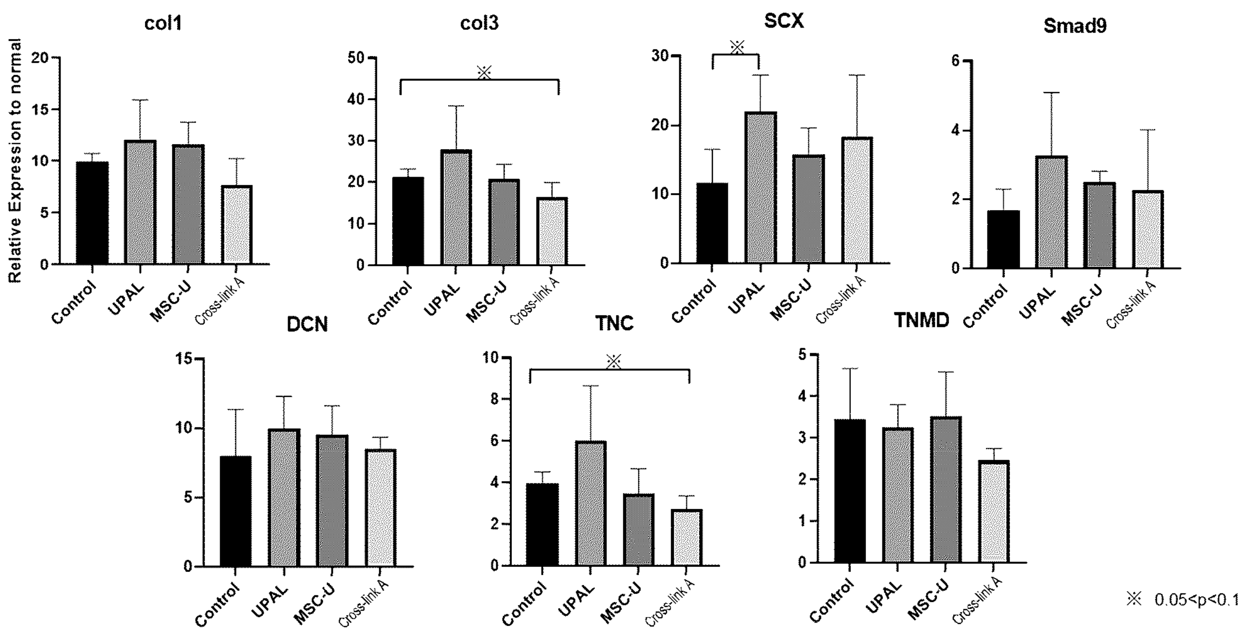
col1
col3
SCX
Smad9
DCN
TNC
TNMD
Relative Expression to normal
Control
UPAL
MSC-U
Cross-link A
※ 0.05<p<0.1

FIG. 11

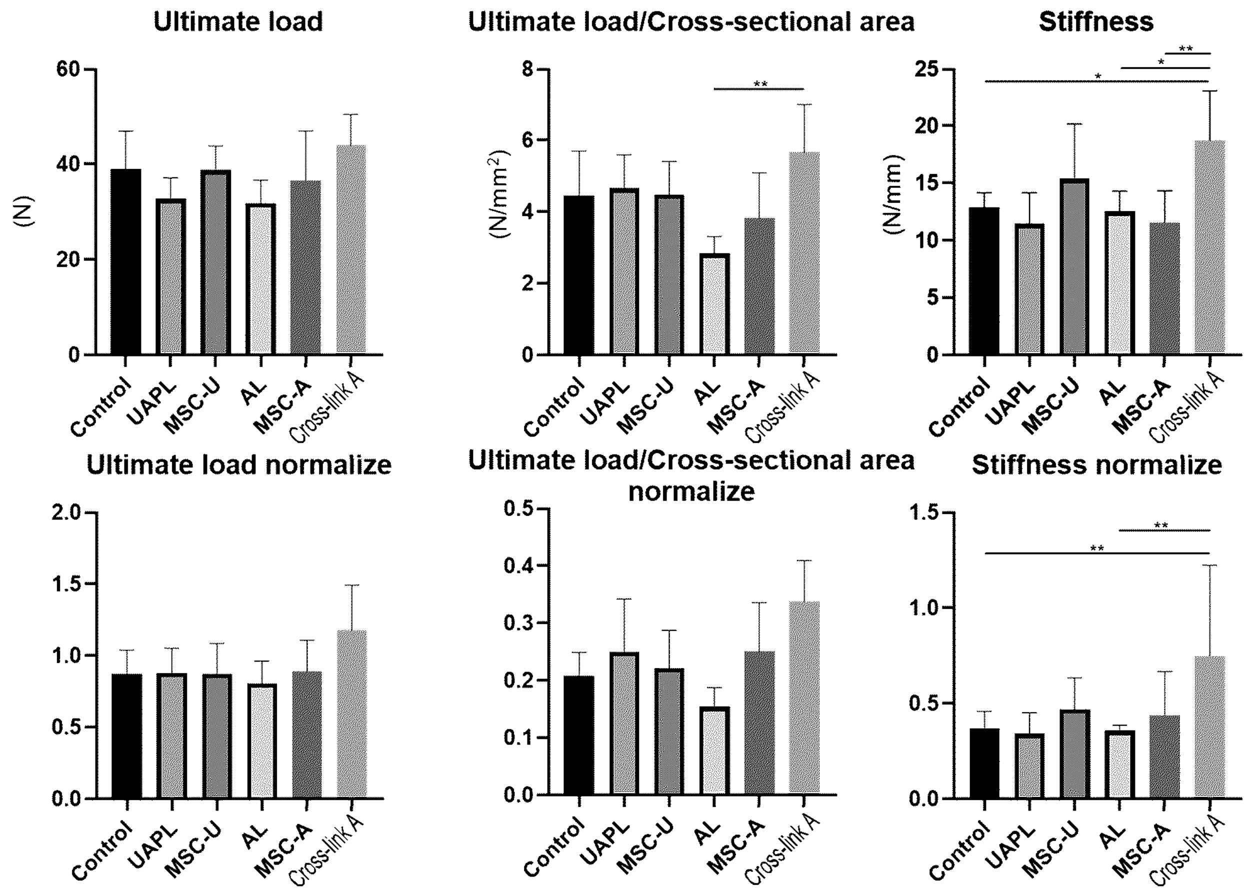
Ultimate load
60
40
20
0
(N)
Ultimate load/Cross-sectional area
8
6
4
2
0
(N/mm2)
**
Stiffness
25
20
15
10
5
0
(N/mm)
*
*
**
Control
UAPL
MSC-U
AL
MSC-A
Cross-link A
Ultimate load normalize
2.0
1.5
1.0
0.5
0.0
Ultimate load/Cross-sectional area normalize
0.5
0.4
0.3
0.2
0.1
0.0
Stiffness normalize
1.5
1.0
0.5
0.0
**
**

**INTERNATIONAL SEARCH REPORT**

International application No. **PCT/JP2022/031816**

**A. CLASSIFICATION OF SUBJECT MATTER**

***A61L 27/20***(2006.01)i; ***A61L 27/38***(2006.01)i; ***A61L 27/52***(2006.01)i
FI: A61L27/20; A61L27/52; A61L27/38 110; A61L27/38

According to International Patent Classification (IPC) or to both national classification and IPC

**B. FIELDS SEARCHED**

Minimum documentation searched (classification system followed by classification symbols)

A61L27/20; A61L27/38; A61L27/52

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | AKTAS, E., et al., Immune modulation with primed mesenchymal stem cells delivered via biodegradable scaffold to repair an Achilles tendon segmental defect, Journal of Orthopaedic Research, 2017, vol. 35, Issue 2, pp. 269-280, ISSN:0736-0266. abstract | 1-19 |
| Y | KIM, Y.-S., et al., Survivorship of implanted bone marrow-derived mesenchymal stem cells in acute rotator cuff tear, Journal of Shoulder and Elbow Surgery, 2013, vol. 22, Issue 8, pp. 1037-1045, ISSN:1058-2746. abstract | 1-19 |
| Y | WO 2020/241904 A1 (KONAN GAKUEN) 03 December 2020 (2020-12-03) claims 1-6, paragraphs [0016]-[0020], [0023]-[0033], [0049]-[0067], [0093]-[0102] | 1-19 |
| Y | NAGAHAMA, K., et al., Living functional hydrogels generated by bioorthogonal cross-linking reactions of azide-modified cells with alkyne-modified polymers, Nature Communications, 2018, vol. 9, Article number: 2195, pp. 1-11, ISSN:2041-1723. abstract, p. 6, right column, line 25 to p. 7, right column, line 8, fig. 5, S10 | 1-19 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **11 October 2022** | **08 November 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

INTERNATIONAL SEARCH REPORT

International application No.
**PCT/JP2022/031816**

**C. DOCUMENTS CONSIDERED TO BE RELEVANT**

| Category* | Citation of document, with indication, where appropriate, of the relevant passages | Relevant to claim No. |
|---|---|---|
| Y | KIMURA, Y., et al., Covalent Cell-Loading Injectable Hydrogel Scaffold Significantly Promotes Tissue Regeneration In Vivo Compared with a Conventional Physical Cell-Loading Hydrogel Scaffold, Advanced Biology, February 2021, vol. 5, Issue 2, Article no. 2000106, pp. 1-10, ISSN:2701-0198. abstract, p. 7, left column, line 3 to p. 9, left column, line 20, fig. 1, scheme S1 | 1-19 |

Form PCT/ISA/210 (second sheet) (January 2015)

**INTERNATIONAL SEARCH REPORT**
**Information on patent family members**

International application No.
**PCT/JP2022/031816**

| Patent document cited in search report | Publication date (day/month/year) | Patent family member(s) | Publication date (day/month/year) |
|---|---|---|---|
| WO 2020/241904 A1 | 03 December 2020 | US 2020/0405914 A1<br>claims 1-6, paragraphs [0055]-[0058], [0062]-[0072], [0090]-[0116], [0153]-[0164] | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2015040276 A
- JP 2020192021 A
- US 20200405914 A
- WO 2020241904 A
- JP 2007075425 A
- WO 2021125255 A

### Non-patent literature cited in the description


- *Nature communications,* 2018, vol. 9, 1-11
- *Advanced Biology,* 2021, vol. 5
- *Journal of orthopaedic research,* February 2017, vol. 35 (2), 269-280
- *Carbohydrate Research,* 1974, vol. 32, 217-225
- **LENNON DP.** Isolation of rat marrow-derived mesenchymal stem cells. *Exp Hematol.,* 2006, vol. 34 (11), 1606-7
- *CHEMICAL ABSTRACTS,* 804514-67-8
- *CHEMICAL ABSTRACTS,* 1255942-08-5
- **ASPENBERG P.** Platelet concentrate injection improves Achilles tendon repair in rats. *Acta Orthop Scand.,* 2004, vol. 75 (1), 93-9
- **IDE J.** The effects of fibroblast growth factor-2 on rotator cuff reconstruction with acellular dermal matrix grafts. *Arthroscopy.,* 2009, vol. 25 (6), 608-16
- **LOPPINI M.** Histopathological scores for tissue-engineered, repaired and degenerated tendon: a systematic review of the literature. *Curr Stem Cell Res Ther.,* 2015, vol. 10 (1), 43-55